# EUROPÄISCHE PATENTSCHRIFT

(11) **EP 1 165 496 B1**
(45) Veröffentlichungstag und Bekanntmachung des Hinweises auf die Patenterteilung: **09.07.2003**
(21) Anmeldenummer: 00917030.9
(22) Anmeldetag: 31.03.2000
(51) Int. Cl.: C07C 251/48, C07C 229/56, C07C 237/30, C07C 255/58, C07D 213/81, C07D 213/89

(54) **SUBSTITUIERTE ANILINVERBINDUNGEN**
SUBSTITUTED ANILINE COMPOUNDS
COMPOSES D'ANILINE SUBSTITUES

(30) Priorität: 31.03.1999 DE 19914721
(43) Veröffentlichungstag der Anmeldung: 02.01.2002
(73) Patentinhaber: BASF AKTIENGESELLSCHAFT, 67056 Ludwigshafen (DE)
(72) Erfinder: HAMPRECHT, Gerhard, D-69469 Weinheim (DE); MENKE, Olaf, D-67317 Altleiningen (DE); PUHL, Michael, D-68623 Lampertheim (DE); REINHARD, Robert, D-67061 Ludwigshafen/Rhein (DE); SAGASSER, Ingo, D-67125 Dannstadt (DE); ZAGAR, Cyrill, D-67061 Ludwigshafen/Rhein (DE)
(74) Vertreter: Kinzebach, Werner, Dr.
(86) Internationale Anmeldenummer: EP0002901
(87) Internationale Veröffentlichungsnummer: WO00059868

(56) Entgegenhaltungen:
- EP-A- 0 257 583
- EP-A- 0 799 825
- DE-A- 2 630 637
- M. ROWLEY ET AL.: "Effect of Plasma Protein Binding on in Vivo Activity and Brain Penetration of Glycine/NMDA Receptor Antagonists" JOURNAL OF MEDICINAL CHEMISTRY, Bd. 40, Nr. 25, 1997, Seiten 4053-4068, XP002144462
- VAIDYA, NITEEN A. ET AL: "Reaction of 2-amino-3-cyano-4-methylfuran as a dienamine and a diene" J. ORG. CHEM., Bd. 47, Nr. 12, 1982, Seiten 2483-2484, XP002144461
- VICTORY P. ET AL.: "A simple synthesis of 2-Methoxypyridine-3-carbonitriles" HETEROCYCLES, Bd. 36, Nr. 4, 1993, XP002144463
- JONES, GRAHAM B. ET AL: "Structurally modified antitumor agents. Part 2. Total synthesis of a cyclopropamitosene" J. CHEM. SOC., PERKIN TRANS. 1 , Bd. 12, 1989, Seiten 2455-2462, XP002144812
- SASAKI K. ET AL.: "Synthesis of Thieno[2,3-h][1,6]naphtyridine from 2-(3-Cyanopropylthio)pyridine-3-carbonitri le: Formation of a Novel Ring System" J. CHEM. SOC., CHEM. COMMUN., Bd. 15, 1994, Seiten 1767-1768, XP002144464 in der Anmeldung erwähnt

## Beschreibung

Die vorliegende Erfindung betrifft mehrfach substituierte Anilinverbindungen sowie ihre Verwendung zur Herstellung neuer, herbizid wirksamer Verbindungen.

Aus Synthesis 1984, 266 ist 2-Aminobenzaldehyd-O-methyloxim bekannt; es wurde nach den in Chem. Ber. 83, 78 (1950) sowie 34, 1330 (1901) beschriebenen Methoden dargestellt und zur Synthese von Dihydrochinolincarbonsäure-Derivaten benutzt.

In der DE 1 231 709 wird ein Verfahren zur Herstellung von o-Aminobenzonitrilen durch Thermolyse von β-Isatinoximen aufgezeigt. Es werden beispielsweise 5-Chlor- und 5-Methyl-2-aminobenzonitril beschrieben, die als Zwischenprodukte für Farbstoffe, Schädlingsbekämpfungsmittel und Heilmittel genutzt werden können.

In der EP 32 672 A wird ein Verfahren zur Herstellung von Anthranilsäureestern durch Umsetzung von Isatinen mit Alkoholen in Gegenwart von Wasserstoffperoxid und Alkalialkoholaten beschrieben. Es wird beispielsweise auf die Synthese von 3,5-Dichloranthranilsäuremethylester eingegangen.

2,3,6-substituierte Aniline sind beschrieben in Chem. Abstr. 73, 3578; 112, 5543; 78, 71690; 118, 147479; 70, 11553; 93, 71617; 121, 255784; 123, 313944; 76, 113245; 79, 146540; 128, 308308 und 131,129760.

Weiterhin sind folgende Einzelverbindungen der Formel I aus der wissenschaftlichen Literatur bekannt:
a) Die Verbindung mit R²¹, R²³ jeweils CN und R²² = Methyl [d. h. die Verbindung 2-Amino-4-methylbenzol-1,3-dicarbonitril] aus J. Org. Chem. 1982, 47, 2483 - 2484 und aus Heterocycles, 36(4), 1993, 769 - 776.
b) Die Verbindung mit R²¹ = CN, R²² = Methyl und R²³ = CO₂CH₃ [d. h. die Verbindung 2-Amino-3-cyano-4-methylbenzoesäuremethylester] aus Heterocycles, 36(4), 1993, 769 - 776.

Die vorliegende Erfindung betrifft substituierte Aniline der Formel 1 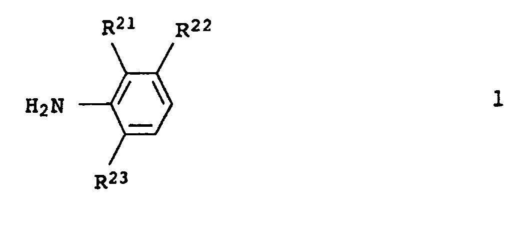 worin die Variablen R²¹, R²² und R²³ die folgenden Bedeutungen haben:
- R²¹: C₁-C₃-Alkoxymethyl, C₁-C₃-Alkoximinomethyl, C₁-C₃-Alkoxycarbonyl, CN, C₁-C₃-Alkyl;
- R²²: Cl, Br, C₁-C₃-Alkyl
- R²³: Wasserstoff, C₁-C₃-Alkoxymethyl, C₁-C₃-Alkoxycarbonyl, CN, Cl, Br, C₁-C₃-Alkyl;
wobei
- mindestens einer der Reste R²¹ oder R²³ für C₁-C₃-Alkoxycarbonyl oder ein funktionelles Derivat davon oder für C₁-C₃-Alkoxymethyl steht;
- R²³ nicht Wasserstoff bedeuten kann und R²² und R²³ nicht die gleiche Bedeutung besitzen, wenn R²¹ für C₁-C₃-Alkoxycarbonyl oder CN steht;
- R²² nicht die gleiche Bedeutung wie R²¹ hat, wenn R²¹ für C₁-C₃-Alkyl und R²³ für C₁-C₃-Alkoxycarbonyl stehen, und R²² nicht Cl bedeutet, wenn R²¹ für CH₃ und R²³ für C₁-C₃-Alkoxycarbonyl stehen;
- R²³ nicht für Wasserstoff steht, wenn R²¹ für C₁-C₃-Alkoxymethyl steht,
und die Salze davon,
ausgenommen die Verbindungen der Formel 1, worin
a) R²¹ und R²³ für CN und R²² für CH₃ steht, und
b) R²¹ für CN, R²² für CH₃ und R²³ für CO₂CH₃ steht.

Es kommen Salze mit anorganischen oder organischen Säuren in Betracht. Geeignete anorganische Säuren sind z.B. Salzsäure, Schwefelsäure, Phosphorsäure, Salpetersäure etc. Geeignete organische Säuren sind z.B. Essigsäure, Citronensäure, Weinsäure etc.

Die bei der Definition von R²¹, R²² und R²³ genannten organischen Molekülteile stellen Sammelbegriffe für individuelle Aufzählungen der einzelnen Gruppenmitglieder dar. Sämtliche Kohlenstoffketten also alle Alkyl-Teile können geradkettig oder verzweigt sein.

Ferner stehen beispielsweise:
- C₁-C₃-Alkoxymethyl für: Methoxymethyl, Ethoxyethyl, n-Propoxymethyl oder 2-Propoxymethyl insbesondere für 2-Methoxymethyl;
- C₁-C₃-Alkoximinomethyl für: Methoximinomethyl, Ethoximinomethyl, n-Propoximinomethyl oder 2-Propoximinomethyl insbesondere für Methoximinomethyl;
- C₁-C₃-Alkoxycarbonyl für: Methoxycarbonyl, Ethoxycarbonyl, n-Propoxycarbonyl oder 2-Propoxycarbonyl, insbesondere für Methoxycarbonyl;
- C₁-C₃-Alkyl für: Methyl, Ethyl, n-Propyl oder 2-Propyl, insbesondere für Methyl oder Ethyl;
- C₁-C₃ Alkoxy für: Methoxy, Ethoxy, n-Propoxy oder 2-Propoxy, insbesondere für Methoxy oder Ethoxy.

Bei dem funktionellen Derivat von C₁-C₃-Alkoxycarbonyl, handelt es sich um CN und C₁-C₃-Alkoximinomethyl.
- R²¹: steht vorzugsweise für Methoxymethyl, Methoximinomethyl, Ethoximinomethyl, Methoxycarbonyl, Ethoxycarbonyl, CN, Methyl oder Ethyl;
- R²²: steht vorzugsweise für Cl, Br, CH₃ oder C₂H₅;
- R²³: steht vorzugsweise für Wasserstoff, Methoxymethyl, Methoxycarbonyl, CN, Cl, Br, Methyl oder Ethyl.

Bevorzugt sind die Verbindungen der Formel 1 worin die Variablen folgende Bedeutung haben:
A)
   - R²¹: für C₁-C₃-Alkoxymethyl, C₁-C₃-Alkoximinomethyl, C₁-C₃-Alkoxycarbonyl, CN oder C₁-C₃-Alkyl;
   - R²²: für Cl, Br oder C₁-C₃-Alkyl und
   - R²³: für C₁-C₃-Alkoxymethyl steht.
B)
   - R²¹: für C₁-C₃-Alkoxymethyl oder C₁-C₃-Alkoximinomethyl;
   - R²²: für Cl, Br oder C₁-C₃-Alkyl und
   - R²³: für H, C₁-C₃-Alkoxymethyl, Cl, Br oder C₁-C₃-Alkyl oder
C)
   - R²¹: C₁-C₃-Alkoximinomethyl;
   - R²²: Cl oder Br;
   - R²³: Wasserstoff, C₁-C₃-Alkoxycarbonyl, oder Cyano.
D)
   - R²¹: C₁-C₃-Alkoxycarbonyl, oder Cyano;
   - R²² und R²³,: die voneinander verschieden sind, Cl, Br oder C₁-C₃-Alkyl, R²³ ferner C₁-C₃-Alkoxycarbonyl.
E)
   - R²¹: C₁-C₃-Alkyl und R²² Cl, Br oder C₁-C₃-Alkyl
   - R²³: C₁-C₃-Alkoxycarbonyl, C₁-C₃-Alkoxymethyl oder Cyano.
F)
   - R²¹: C₁-C₃-Alkoxymethyl oder C₁-C₃-Alkoximinomethyl
   - R²²: Cl, Br oder C₁-C₃-Alkyl
   - R²³: C₁-C₃-Alkoxycarbonyl, CN oder C₁-C₃-Alkyl.

Die Verbindungen der Formel 1 sind Zwischenprodukte zur Herstellung der herbizid wirksamen Verbindungen der Formel A 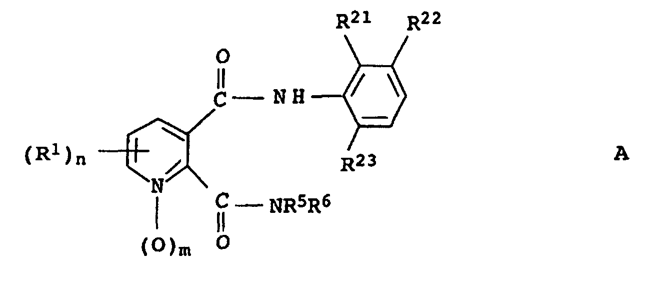 worin die Variablen R²¹, R²² und R²³ die folgenden Bedeutungen haben:
- R²¹: C₁-C₃-Alkoxymethyl, C₁-C₃-Alkoximinomethyl, C₁-C₃-Alkoxycarbonyl, CN, C₁-C₃-Alkyl;
- R²²: Cl, Br, C₁-C₃-Alkyl
- R²³: Wasserstoff, C₁-C₃-Alkoxymethyl, C₁-C₃-Alkoxycarbonyl, CN, Cl, Br, C₁-C₃-Alkyl;
wobei
- mindestens einer der Reste R²¹ oder R²³ für C₁-C₃-Alkoxycarbonyl oder ein funktionelles Derivat davon oder für C₁-C₃-Alkoxymethyl steht;
- R²³ nicht Wasserstoff bedeuten kann und R²² und R²³ nicht die gleiche Bedeutung besitzen, wenn R²¹ für C₁-C₃-Alkoxycarbonyl oder CN steht;
- R²² nicht die gleiche Bedeutung wie R²¹ hat, wenn R²¹ für C₁-C₃-Alkyl und R²³ für C₁-C₃-Alkoxycarbonyl stehen, und R²² nicht Cl bedeutet, wenn R²¹ für CH₃ und R²³ für C₁-C₃-Alkoxycarbonyl stehen;
- R²³ nicht für Wasserstoff steht, wenn R²¹ für C₁-C₃-Alkoxymethyl steht,
ausgenommen die Verbindungen der Formel 1, worin
a) R²¹ und R²³ für CN und R²² für CH₃ steht, und
b) R²¹ für CN, R²² für CH₃ und R²³ für CO₂CH₃ steht,
   - R¹: Halogen, CN, NO₂, C₁-C₃-Alkyl, C₁-C₃-Haloalkyl, C₁-C₃-Alkoxy, C₁-C₃-Haloalkoxy, C₁-C₃-Alkylthio, C₁-C₃-Haloalkylthio, C₁-C₃-Alkylsulfinyl, C₁-C₃-Haloalkylsulfinyl, C₁-C₃-Alkylsulfonyl, C₁-C₃-Haloalkylsulfonyl, C₃-C₆-Cycloalkyl, C₃-C₆-Cycloalkyl-C₁-C₃-alkyl, C₂-C₄-Alkenyl, C₂-C₄-Haloalkenyl, C₂-C₄-Alkinyl, C₃-C₄-Haloalkinyl, Amino, C₁-C₃-Monoalkylamino oder C₁-C₃-Alkylcarbonyl;
   - R⁵: Wasserstoff, C₁-C₃-Alkyl, OH oder C₁-C₄-Alkoxy;
   - R⁶: Wasserstoff, C₁-C₆-Alkyl, C₁-C₆-Haloalkyl, C₁-C₆-Cyanoalkyl, C₃-C₆-Cycloalkyl, C₃-C₆-Cycloalkenyl, C₃-C₆-Cycloalkyl-C₁-C₃-alkyl, C₃-C₆-Cycloalkyl, das 1 oder 2 Substituenten aufweist, die unabhängig voneinander ausgewählt sind unter Halogen oder C₁-C₃-Alkyl, C₃-C₆-cycloalkoxy-C₁-C₃-alkyl, C₁-C₆-Alkoxy-C₁-C₆-alkyl, C₁-C₆-Alkylthio-C₁-C₆-alkyl, C₁-C₆-Alkoxycarbonyl-C₁-C₆-alkyl, C₃-C₆-Alkenyl, C₃-C₆-Alkinyl, Amino, C₁-C₄-Monoalkylamino, Di-C₁-C₄-alkylamino oder R⁶ zusammen mit R⁵ ein 5- oder 6-gliedriger Heterocyclus, der 1, 2 oder 3 Heteroatome, die unabhängig voneinander ausgewählt sind unter N, O und S, und gegebenenfalls 1 oder 2 Substituenten aufweist, die unabhängig voneinander ausgewählt sind unter Halogen oder C₁-C₃-Alkyl, C₁-C₃-Alkoxy oder C₁-C₃-Haloalkyl;

m für 0 oder 1 steht und n für 0, 1, 2 oder 3 steht. Die Verbindungen der Formel A sind Gegenstand der DE 199 14 721 vom 31.3.1999 und WO- 00/58288.

Beispiele für bevorzugte substituierte Aniline der Formel I sind in Tabelle 1 angegeben, worin R²¹ bis R²³ folgende Bedeutungen aufweisen:

| Nr. | R²¹ | R²² | R²³ |
|---|---|---|---|
| 1 | CH=NOCH₃ | Cl | H |
| 2 | CH=NOCH₃ | Br | H |
| 3 | CH=NOCH₃ | CH₃ | H |
| 4 | CH=NOCH₃ | C₂H₅ | H |
| 5 | CH=NOCH₃ | Cl | CO₂CH₃ |
| 6 | CH=NOCH₃ | Br | CO₂CH₃ |
| 7 | CH=NOCH₃ | CH₃ | CO₂CH₃ |
| 8 | CH=NOCH₃ | Cl | CN |
| 9 | CH=NOCH₃ | Br | CN |
| 10 | CH=NOCH₃ | Cl | Cl |
| 11 | CH=NOCH₃ | Cl | Br |
| 12 | CH=NOCH₃ | Br | Cl |
| 14 | CH=NOCH₃ | Cl | CH₃ |
| 15 | CH=NOCH₃ | Br | CH₃ |
| 16 | CH=NOCH₃ | CH₃ | CN |
| 17 | CH=NOCH₃ | CH₃ | Cl |
| 22 | CH=NOCH₃ | Cl | C₂H₅ |
| 23 | CH=NOCH₃ | Br | C₂H₅ |
| 24 | CH=NOC₂H₅ | Cl | H |
| 25 | CH=NOC₂H₅ | Br | H |
| 26 | CH=NOC₂H₅ | CH₃ | H |
| 27 | CH=NOC₂H₅ | C₂H₅ | H |
| 28 | CH=NOC₂H₅ | Cl | CO₂CH₃ |
| 29 | CH=NOC₂H₅ | Br | CO₂CH₃ |
| 30 | CH=NOC₂H₅ | CH₃ | CO₂CH₃ |
| 31 | CH=NOC₂H₅ | Cl | CN |
| 32 | CH=NOC₂H₅ | Br | CN |
| 33 | CH=NOC₂H₅ | Cl | Cl |
| 34 | CH=NOC₂H₅ | Cl | Br |
| 35 | CH=NOC₂H₅ | Br | Cl |
| 37 | CH=NOC₂H₅ | Cl | CH₃ |
| 38 | CH=NOC₂H₅ | Br | CH₃ |
| 39 | CH=NOC₂H₅ | CH₃ | CN |
| 40 | CH=NOC₂H₅ | CH₃ | Cl |
| 45 | CH=NOC₂H₅ | Cl | C₂H₅ |
| 46 | CH=NOC₂H₅ | Br | C₂H₅ |
| 47 | CO₂CH₃ | Cl | Br |
| 48 | CO₂CH₃ | Cl | CH₃ |
| 49 | CO₂CH₃ | Br | Cl |
| 50 | CO₂CH₃ | CH₃ | Cl |
| 51 | CO₂CH₃ | CH₃ | Br |
| 52 | CO₂CH₃ | CH₃ | CO₂CH₃ |
| 54 | CO₂CH₃ | Cl | CO₂CH₃ |
| 57 | CO₂CH₃ | Cl | CN |
| 58 | CO₂CH₃ | Br | CO₂CH₃ |
| 62 | CO₂CH₃ | Br | CN |
| 64 | CO₂CH₃ | Br | CH₃ |
| 65 | CO₂CH₃ | Cl | C₂H₅ |
| 66 | CO₂CH₃ | Br | C₂H₅ |
| 68 | CO₂CH₃ | CH₃ | CN |
| 71 | CO₂C₂H₅ | Cl | Br |
| 72 | CO₂C₂H₅ | Cl | CH₃ |
| 73 | CO₂C₂H₅ | Br | Cl |
| 74 | CO₂C₂H₅ | CH₃ | Cl |
| 75 | CO₂C₂H₅ | CH₃ | Br |
| 76 | CO₂C₂H₅ | CH₃ | CO₂CH₃ |
| 78 | CO₂C₂H₅ | Cl | CO₂CH₃ |
| 81 | CO₂C₂H₅ | Cl | CN |
| 82 | CO₂C₂H₅ | Br | CO₂CH₃ |
| 86 | CO₂C₂H₅ | Br | CN |
| 88 | CO₂C₂H₅ | Br | CH₃ |
| 89 | CO₂C₂H₅ | Cl | C₂H₅ |
| 90 | CO₂C₂H₅ | Br | C₂H₅ |
| 92 | CO₂C₂H₅ | CH₃ | CN |
| 143 | CN | Cl | Br |
| 144 | CN | Cl | CH₃ |
| 145 | CN | Br | Cl |
| 146 | CN | CH₃ | Cl |
| 147 | CN | CH₃ | Br |
| 148 | CN | CH₃ | CO₂CH₃ |
| 150 | CN | Cl | CO₂CH₃ |
| 153 | CN | Cl | CN |
| 154 | CN | Br | CO₂CH₃ |
| 158 | CN | Br | CN |
| 160 | CN | Br | CH₃ |
| 161 | CN | Cl | C₂H₅ |
| 162 | CN | Br | C₂H₅ |
| 164 | CN | CH₃ | CN |
| 173 | CH₃ | Br | CO₂CH₃ |
| 176 | C₂H₅ | Cl | CO₂CH₃ |
| 177 | C₂H₅ | Br | CO₂CH₃ |
| 180 | CH₃ | CH₃ | CN |
| 184 | CH₃ | Cl | CN |
| 185 | CH₃ | Br | CN |
| 186 | C₂H₅ | Cl | CN |
| 187 | C₂H₅ | Br | CN |
| 190 | C₂H₅ | CH₃ | CN |
| 192 | CH₃OCH₂ | Cl | CH₃ |
| 193 | CH₃OCH₂ | CH₃ | Cl |
| 194 | CH₃OCH₂ | CH₃ | CH₃ |
| 195 | CH₃OCH₂ | Cl | Cl |
| 196 | CH₃OCH₂ | Br | CH₃ |
| 197 | CH₃OCH₂ | Cl | C₂H₅ |
| 198 | CH₃OCH₂ | Br | C₂H₅ |
| 199 | CH₃ | Cl | CH₃OCH₂ |
| 200 | C₂H₅ | Cl | CH₃OCH₂ |
| 201 | CH₃ | Br | CH₃OCH₂ |
| 202 | C₂H₅ | Br | CH₃OCH₂ |
| 203 | CH₃OCH₂ | CH₃ | CO₂CH₃ |
| 204 | CH₃OCH₂ | Cl | CO₂CH₃ |
| 205 | CH₃OCH₂ | Br | CO₂CH₃ |
| 208 | CH₃OCH₂ | Cl | CN |
| 209 | CH₃OCH₂ | Br | CN |
| 210 | CO₂CH₃ | Cl | CH₃OCH₂ |
| 212 | CN | Cl | CH₃OCH₂ |
| 213 | CN | Br | CH₃OCH₂ |

Die Aniline der Formel 1 sind nach einem der folgenden Verfahren herstellbar. Die dabei als Einsatzstoffe verwendeten Isatosäureanhydride und deren Herstellung sind an sich bekannt und in der Literatur, z.B. Chem. Abstr. 75, 98482; 117, 233811; 125, 300514, beschrieben. Das 3-Chlor-6-methyl-isatosäureanhydrid wird bei der Synthese der Ausgangsstoffe näher beschrieben.

Die substituierten Aniline der allgemeinen Formel 1a, in der R²¹ für einen C₁-C₃-Alkoximinomethylrest steht, können hergestellt werden, indem man beispielsweise einen substituierten o-Nitrobenzaldehyd der Formel 2 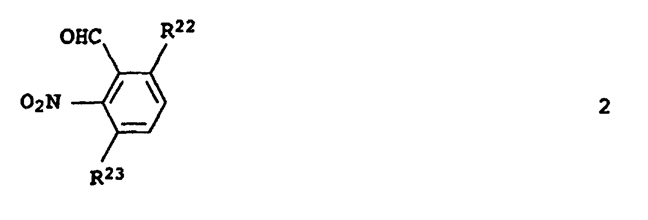 mit Hydroxylaminhydrochlorid in Gegenwart einer Base umsetzt und das so erhaltene Oxim der Formel 4 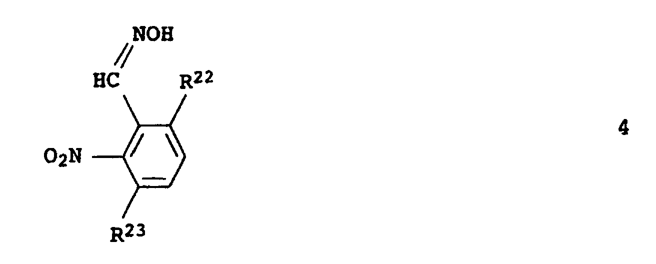 mit einem Alkylierungsmittel der Formel RG, worin R für einen C₁-C₃-Alkylrest steht und G eine nucleophil verdrängbare Abgangsgruppe bedeutet, in Gegenwart einer Base umsetzt und den so erhaltenen Oximether der Formel 6 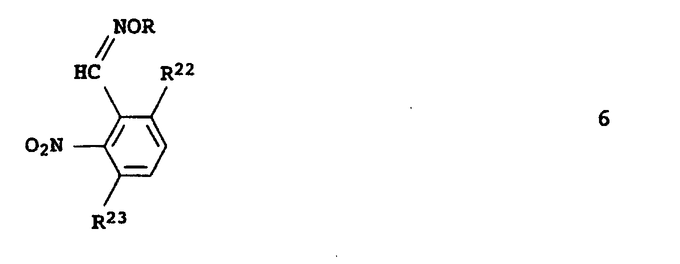 reduziert, z.B. mit Eisen in Gegenwart einer Säure oder mit Wasserstoff in Gegenwart eines Metallkatalysators.

Beispiele für geeignete, nucleophil verdrängbare Abgangsgruppen sind Halogen, vorzugsweise Chlor, Brom oder Jod, C₁-C₃-Alkylsulfonyloxy wie Methylsulfonyloxy, Phenylsulfonyloxy, worin der Phenylrest gegebenenfalls mit Halogen oder C₁-C₆-Alkyl ein- oder mehrfach substituiert sein kann, wie Phenylsulfonyloxy, p-Toluolsulfonyloxy oder p-Cl-Phenylsulfonyloxy oder ein C₁-C₃-Dialkylsulfat wie Dimethyl- oder Diethylsulfat.

Man kann die Oximether der Formel 6 jedoch auch durch direkte Oximierung eines substituierten o-Nitrobenzaldehyds 2 mit einem C₁-C₃-Alkoxyamin der Formel 7

H₂NOAlk 7

oder einem Salz davon, z.B. dem Hydrochlorid, in Gegenwart einer Base herstellen und dann wie oben zu dem Anilin 1 reduzieren.

Die substituierten Aniline der Formel 1b, in der einer der Reste R²¹ oder R²³ für eine Carbonsäurefunktion steht, werden ebenfalls nach an sich bekannten Verfahren hergestellt, indem man beispielsweise ein substituiertes Anilin der Formel 8a oder 8b 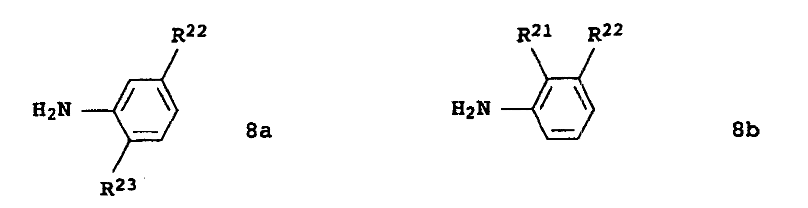 mittels Chloralhydrat und Hydroxylaminsulfat in die entsprechenden Isonitrosoacetanilide der Formeln 9a oder 9b 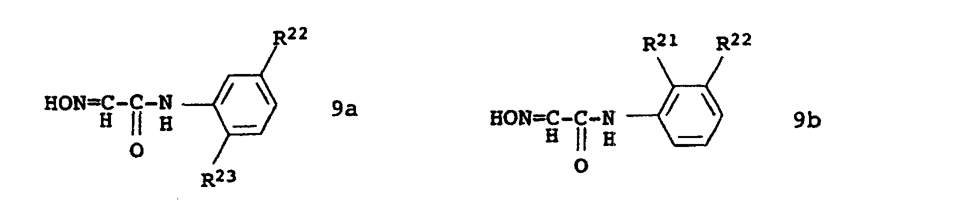 umwandelt, diese in Gegenwart einer Säure, z.B. Schwefelsäure zu den entsprechenden Isatinen der Formeln 10a oder 10b 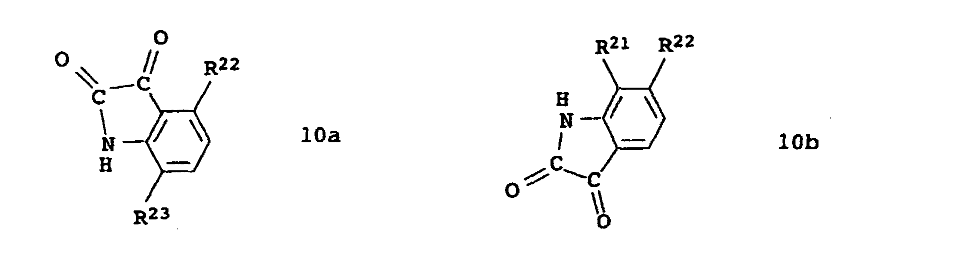 cyclisiert und letztere mit C₁-C₃-Alkanolen in Gegenwart von Alkali-C₁-C₃-Alkanolaten und von wässrigem Wasserstoffperoxid zu den entsprechenden Anthranilestern der Formeln 13a oder 13b umsetzt.

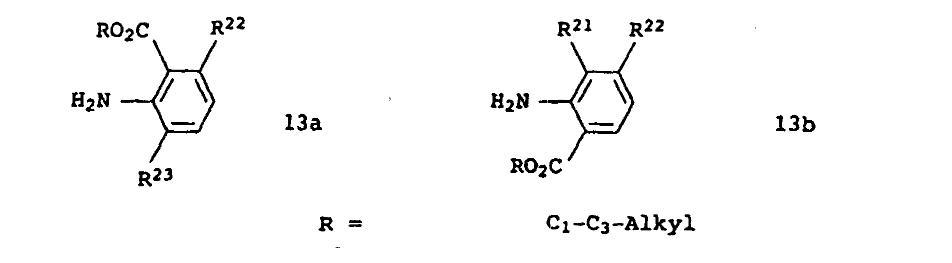

Durch Umesterung mit höheren Alkoholen können diese wiederum in entsprechende längerkettige Anthranilester umgewandelt werden.

Setzt man die Isatine der Formeln 10a oder 10b mit Hydroxylamin wie für die Oxime 4 beschrieben um, so erhält man die Isatin-β-oxime der Formeln 14a oder 14b, 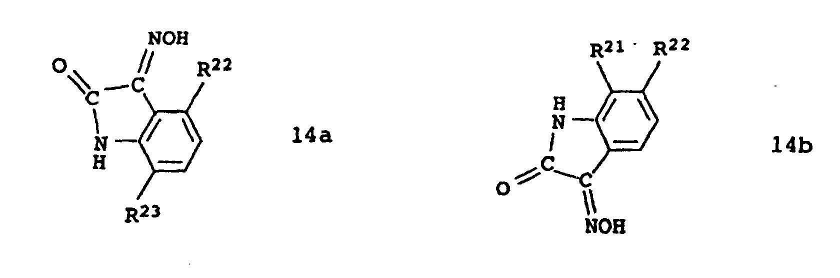 die durch Erhitzen unter vermindertem Druck in Gegenwart von inerten Lösungsmitteln, deren Siedepunkte nicht unter dem Siedepunkt der entstehenden Spaltprodukte liegen, z.B. Diethylglycolether, Diethylenglycoldimethylether, Tetraethylharnstoff, Tetrabutylharnstoff, Dimethylethylenharnstoff, Dimethylpropylenharnstoff, Phthalsäurediethylether, Phthalsäurediethylhexylester, Octaethylenglycol oder Nonaethylenglycol, zu den entsprechenden o-Amino-benzonitrilen der Formeln 15a oder 15b 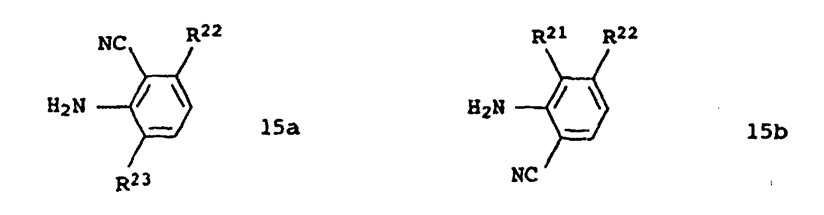 umgesetzt werden.

Nach einem weiteren Verfahren kann man die Isatine der Formeln 10a oder 10b auch mit wässrigem Wasserstoffperoxid in einer aliphatischen Carbonsäure, wie Eisessig, in Gegenwart von konz. Schwefelsäure zu den Isatosäureanhydriden der Formeln 16a oder 16b 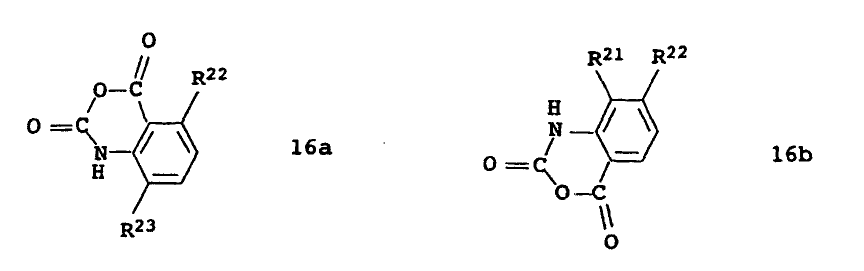 umsetzen.

Letztere lassen sich analog zu der entsprechenden Umsetzung der Isatine 10a oder 10b mit C₁-C₃-Alkanolen in Gegenwart einer Base in die Anthranilester der Formeln 13a oder 13b überführen.

Verwendet man als Nucleophil Ammoniak in wässriger Lösung, erhält man stattdessen die Carbamoylderivate 17a oder 17b.

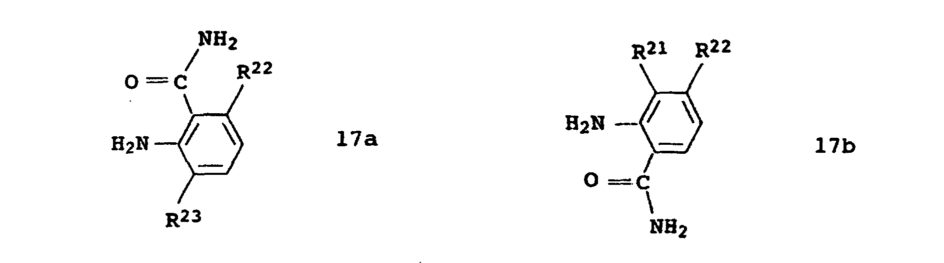

Diese lassen sich - zweckmäßig nach Überführung in entsprechende Mineralsäuresalze - durch wasserentziehende Mittel, z.B. Phosphoroxychlorid, in die entsprechenden Nitrile der Formeln 15a oder 15b 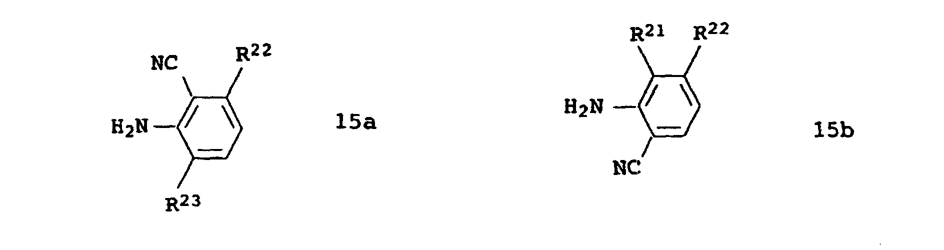 umwandeln.

Für die Herstellung der Verbindungen laz, in der R²¹ einen Methoximinomethylrest bedeutet, steht beispielhaft die im folgenden Schema 1 beschriebene Umsetzung, wobei ausgehend von einem substituierten o-Nitrobenzaldehyd 2z durch Umsetzung mit Hydroxylaminhydrochlorid in Gegenwart einer Base und nachfolgende Methylierung, z.B. mit Methyljodid, der Oximether 6z erhalten wird. Dieser kann auch durch direkte Umsetzung mit O-Methylhydroxylamin-hydrochlorid 7 in Gegenwart einer Base gewonnen und dann zu dem Anilinderivat laz reduziert werden, z.B. durch Reduktion mit Eisen.

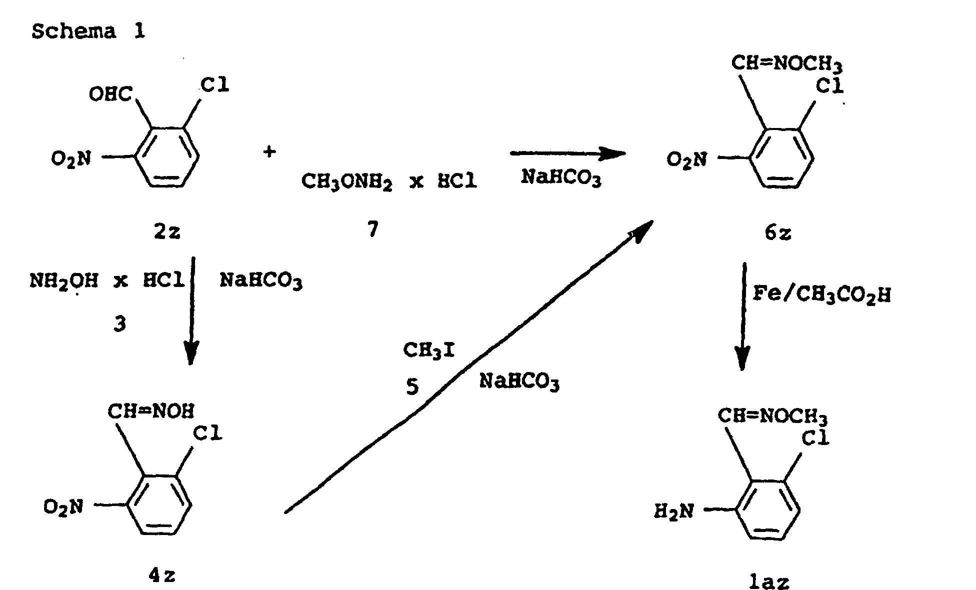

Methoden zur Herstellung von Oximethern sind in Synthesis 1984, 266, Chem. Ber. 83, 78 (1950) und 34, 1330 (1901) beschrieben.

zur Herstellung der als weitere Ausgangsmaterialien benötigten rsatine, z.B. der Isatine 10az oder 10bz, setzt man substituierte Aniline 8az oder 8bz mit Chloralhydrat und Hydroxylamin zu den entsprechenden Isonitrosoacetaniliden 9az oder 9bz um und cyclisiert diese mit Schwefelsäure gemäß folgendem Schema 2. Die Synthese von Isatinen ist beispielsweise in Beilstein, 21, I 402-405 und 21, IV 5451 beschrieben.

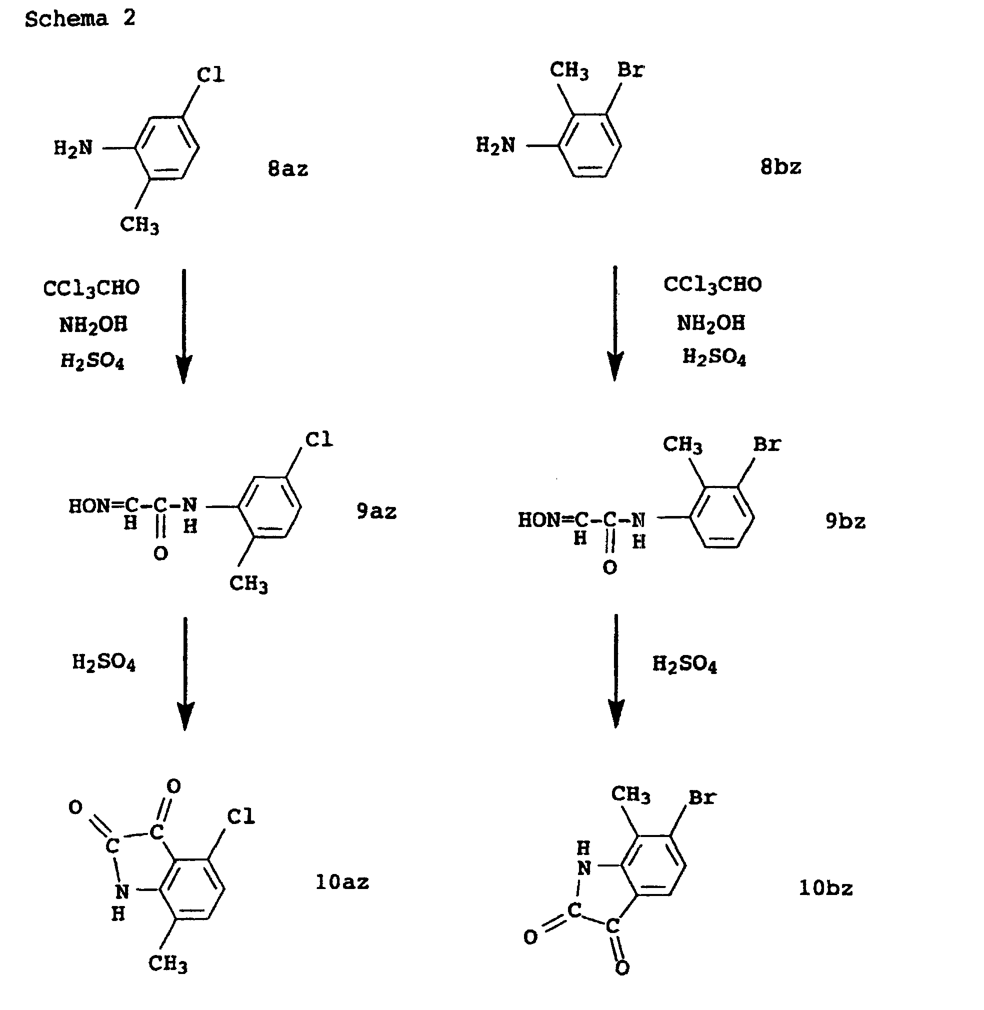

Die Isatine 10az oder 10bz lassen sich beispielsweise durch Reaktion mit Methanol in Gegenwart von Natriummethylat und wässrigem Wasserstoffperoxid in Anthranilsäuremethylester 13az oder 13bz gemäß Schema 3 umsetzen. Das Verfahren ist in der EP 32672 A beschrieben. Man kann die Isatine 10az oder 10bz ferner auch nach JP-A-62234080 in ihre β-Oxime umwandeln und diese durch Erhitzen unter vermindertem Druck nach dem Verfahren der DE 1231 709 in substituierte o-Aminobenzonitrile 15az oder 15bz überführen, siehe ebenfalls Schema 3.

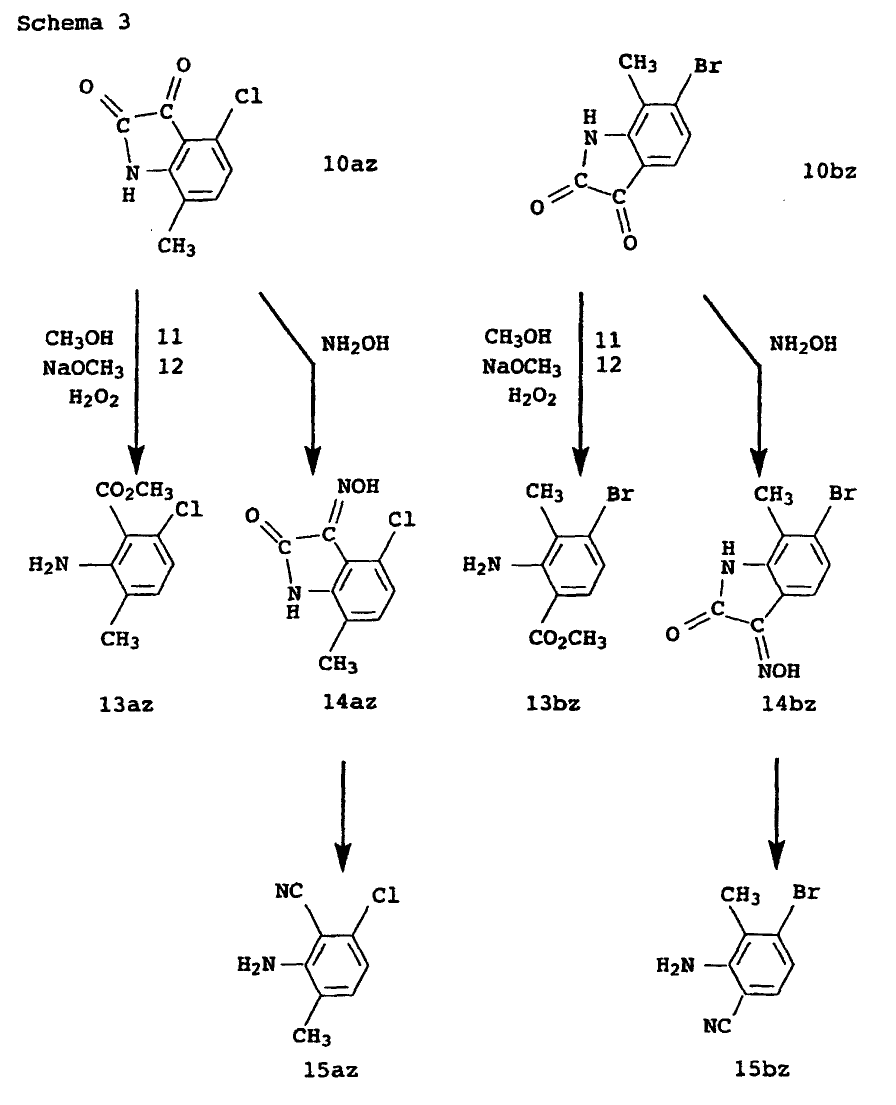

Nach einem alternativen Verfahren kann man gemäß Angew. Chem. 1980, 92, 196 die Isatine 10az oder 10bz jedoch auch zuerst mit wässrigem Wasserstoffperoxid in einer aliphatischen Carbonsäure, wie Eisessig, in Gegenwart von konz. Schwefelsäure zu den Isatosäureanhydriden 16az oder 16bz gemäß Schema 4 umsetzen. Letztere ergeben bei der Umsetzung mit Alkoholen in Gegenwart einer Base, beispielsweise Triethylamin, die Anthranilester 13az oder 13bz. Bei der Umsetzung mit wässrigem Ammoniak erhält man nach Schema 4 die Carbamoylderivate 17az oder 17bz. Diese lassen sich zweckmäßig in Form ihrer Salze - beispielsweise als Hydrochloride - mit wasserentziehenden Mitteln, wie Phosphoroxychlorid, in die entsprechenden Nitrile 15az oder 15bz umwandeln.

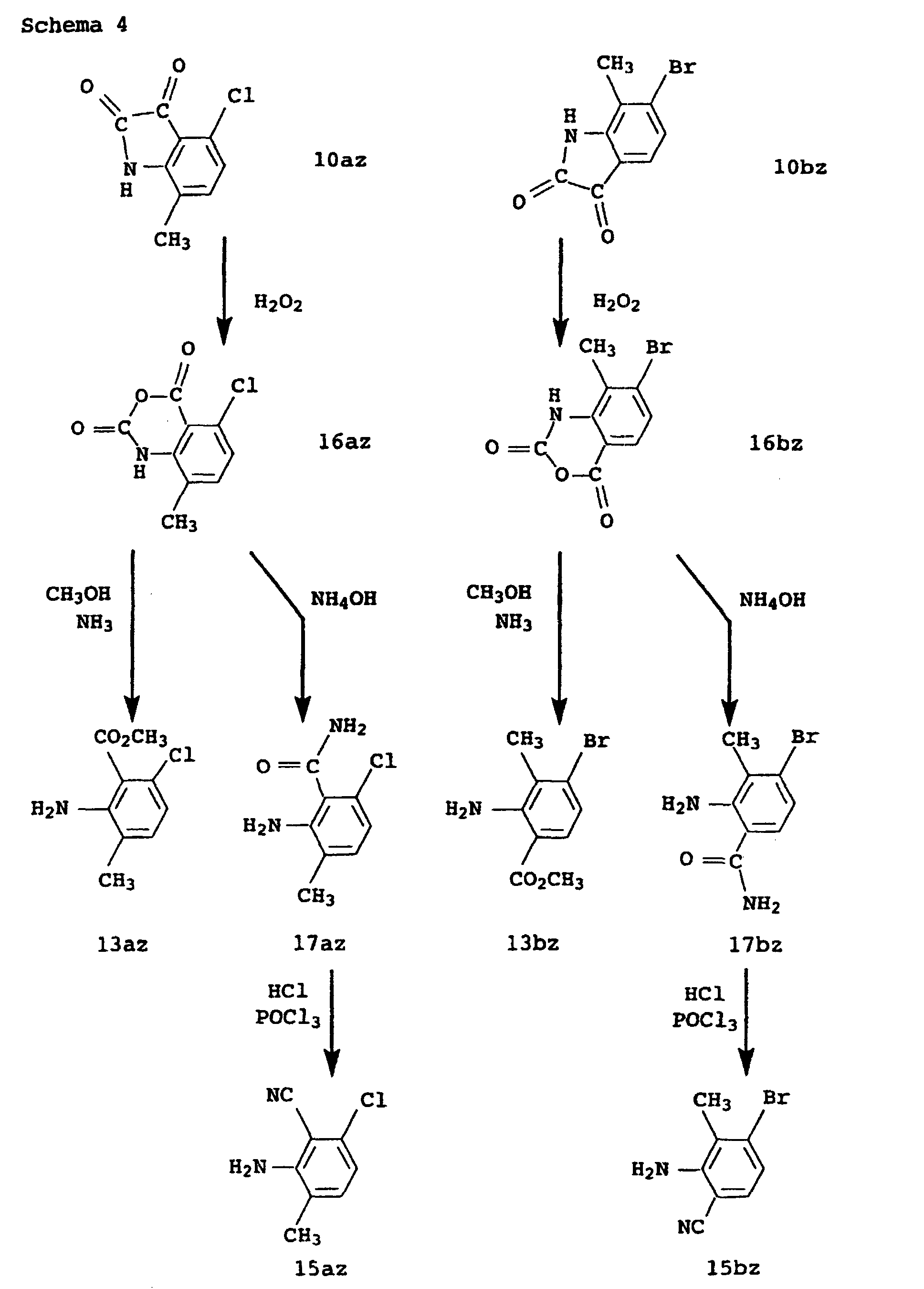

Die Ringöffnung von Isatosäureanhydriden mit Alkoholen ist in J. Med. Chem. 1988, 31, 2136 und in WO 97/08130 beschrieben. Verwendet man als Nucleophil für die Ringöffnung wässriges Ammoniak, so kann man nach der Verfahrensweise der DE 15 43 332 arbeiten. Die Dehydratisierung der Carbamoylderivate zu entsprechenden Benzonitrilen kann man nach den Verfahrensweisen von J. Chem. Soc. Chem. Commun. 1994, (15), 1767 und J. Heterocycl. Chem. 1997, 34, 1661 durchführen.

3-Chlor-2-methoxymethyl-anilin ist aus EP 127 114 und DE 23 45 443 bekannt; es kann nach den Schemata 2-4 in entsprechende 6-substituierte Carbonsäureabkömmlinge umgewandelt werden.

Daneben kann man jedoch auch Anthranilester der Formel 13a oder 13b 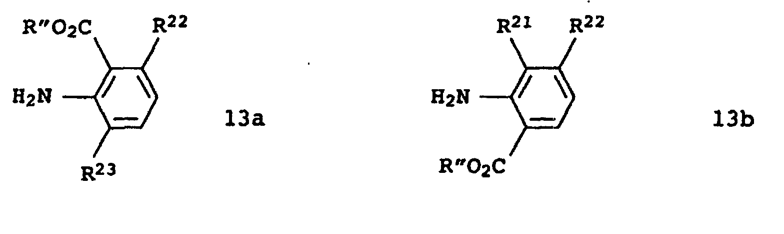 in der R" beispielsweise für einen C₁-C₃-Alkylrest steht und R²¹ C₂-C₃-Alkyl, R²² und R²³ Cl, Br, C₂-C₃-Alkyl, R²¹ und R²³ ferner CN oder C₁-C₃-Alkoxycarbonyl bedeuten, beispielsweise nach Schutz der Aminogruppe mit einem Acylierungsagens 18

R'C(O)G 18

in dem R' einen C₁-C₄-Alkylrest bedeutet und G für eine nucleophil verdrängbare Abgangsgruppe steht (Beispiele wurden bereits oben genannt), zu den Acylanilinen 19a oder 19b 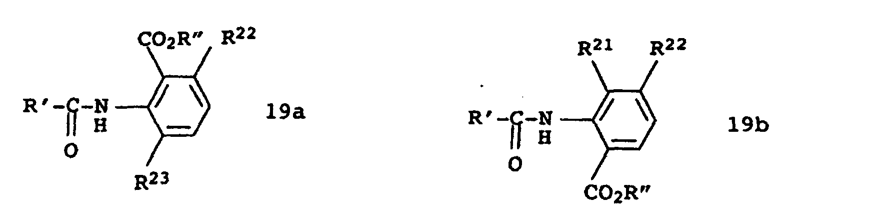 umsetzen, diese mit einem komplexen Metallhydrid 20

Me^{I}Me^{III}H₄ 20

in der Me^{I} bzw. Me^{III} jeweils für Metalle der 1. oder 3. Hauptgruppe stehen, zu den Benzylalkoholen 21a oder 21b reduzieren, 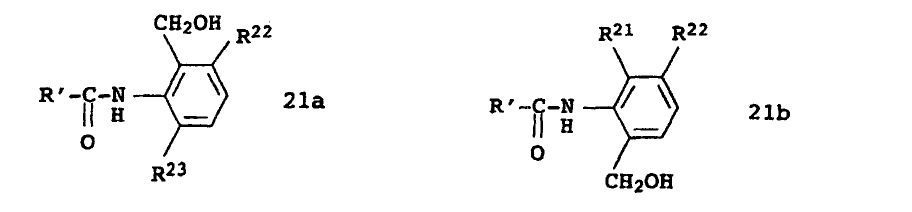 diese mit einem Alkylierungsmittel RG (R = C₁-C₃-Alkyl) in Gegenwart einer Base zu den Alkoxymethylvertretern 22a oder 22b umsetzen 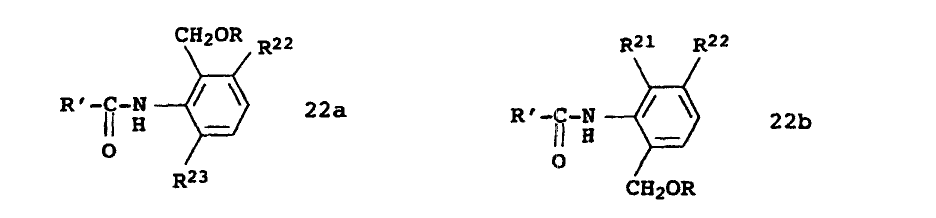 und letztere in Gegenwart von wässrigem Alkali oder Mineralsäure unter Abspaltung der Schutzgruppe zu den freien Anilinen 23a oder 23b umsetzen.

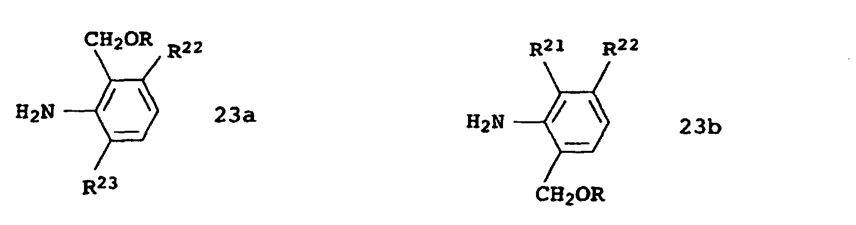

Statt der Anthranilester 13a oder 13b kann man auch die entsprechenden Anthranilsäuren für die Reduktion verwenden.

Im Falle der Verwendung der Aniline 13az oder 13bz, in denen R²³ bzw. R²¹ und R" Methyl bedeuten und R²² für Cl steht, erfolgt die Herstellung beispielsweise von Methoxymethylvertretern 23az oder 23bz nach dem Schema 5:

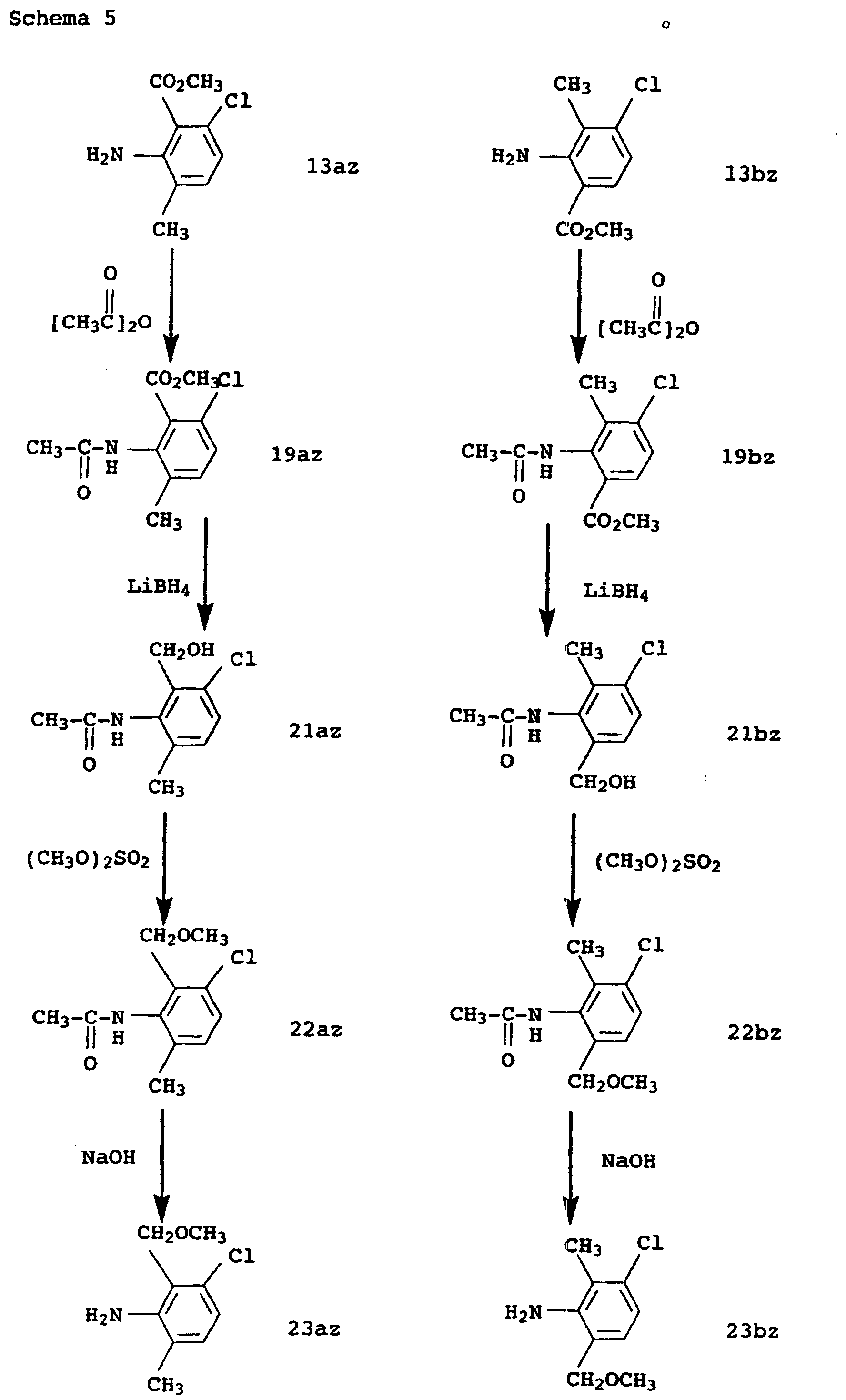

Man kann jedoch auch die Acylaniline der Formeln 19c oder 19d 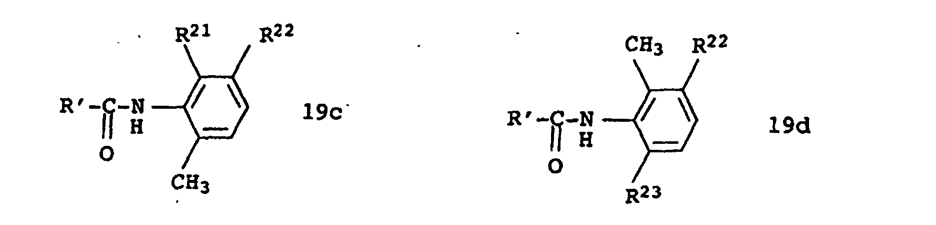 in der R²¹ CN oder C₁-C₃-Alkoxycarbonyl und R²³ Cl, Br, CN oder C₁-C₃-Alkoxycarbonyl und R²² Cl oder Br bedeuten, an der Tolylseitenkette zu den Benzylhalogeniden 24c oder 24d, in denen Hal Cl oder Br bedeutet, halogenieren 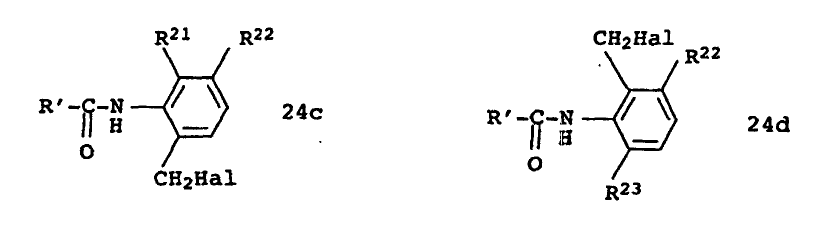 und diese mit einem Alkohol R'"OH oder Alkoholat R'"OMe, worin R'" für einen C₁-C₃-Alkylrest und Me für ein Alkali- oder Erdalkalimetallatom steht, gegebenenfalls in Gegenwart einer Base, zu den Benzylethern 22c oder 22d 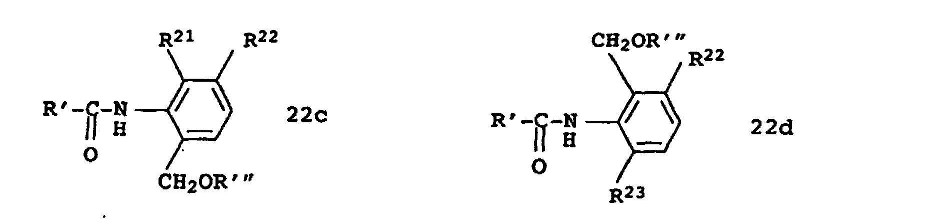 umsetzen und diese in Gegenwart von wässrigem Alkali oder verdünnter Schwefel-, Salz- oder Phosphorsäure zu den freien Anilinen 23c oder 23d spalten.

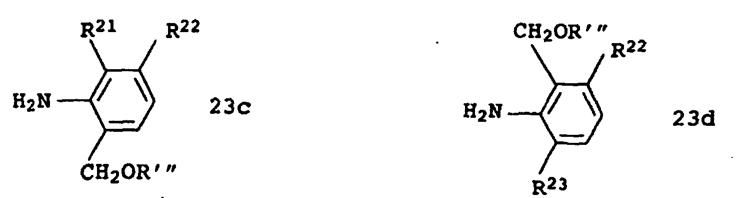

Im Falle der Verwendung der Acylaniline 19cz oder 19dz, in denen R²¹ und R²³ CN bedeuten und R²² für Cl steht, erfolgt die Herstellung beispielsweise der Methoxymethylvertreter 23cz oder 23dz nach dem Schema 6: 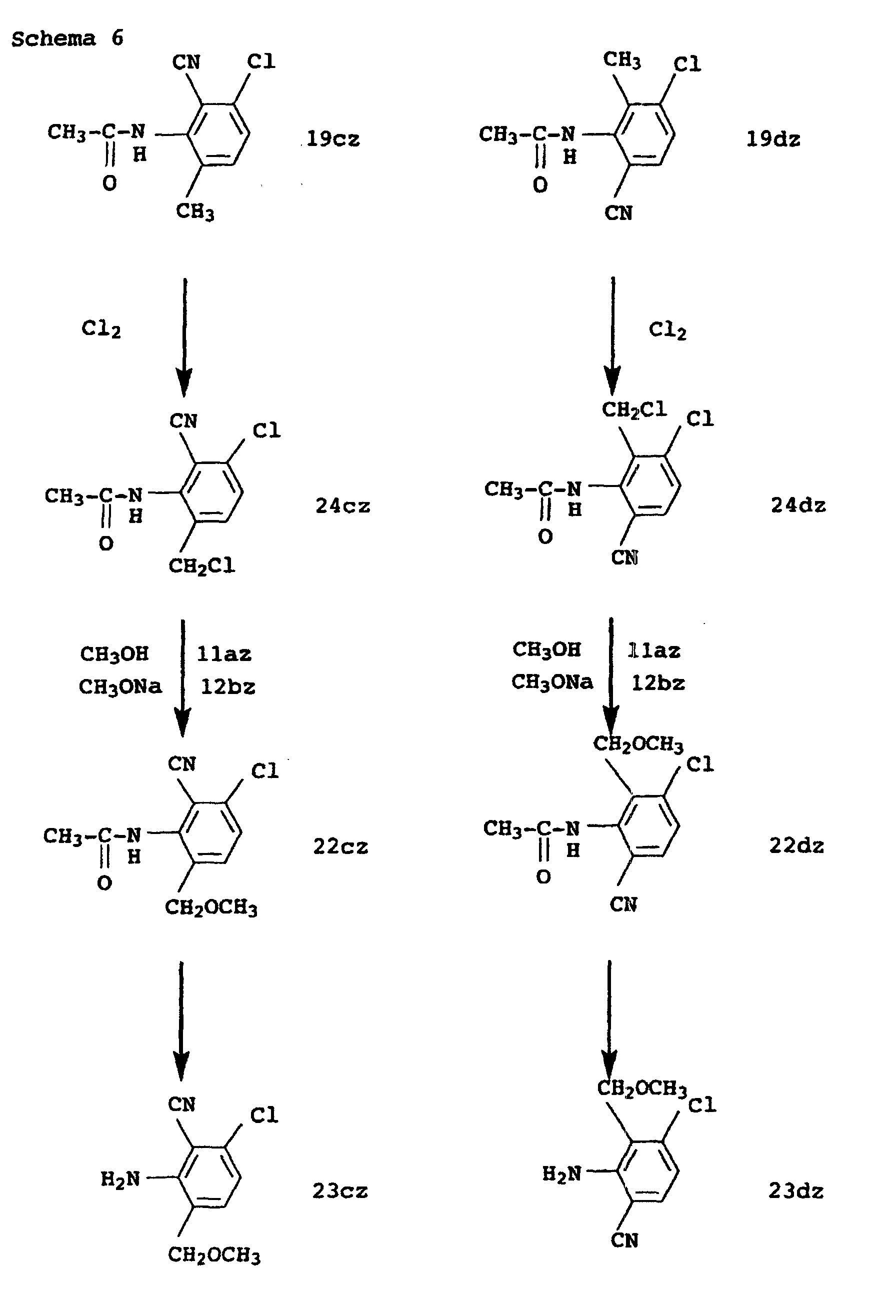 Verwendet man statt der Nitrile 19cz oder 19dz entsprechende Anthranilester, so werden diese bei der Abspaltung der Acylaminogruppe in der Regel ebenfalls verseift, so dass sie nachträglich wieder verestert werden müssen, beispielsweise durch Rückflußkochen in alkoholischer Salzsäure unter Durchleiten von Chlorwasserstoffgas.

Im Folgenden werden die Reaktionsschritte des Schemas 1 näher erläutert. Durch Zugabe eines Alkali- oder Erdalkalihydrogencarbonats zu der wässrigen Lösung eines O-Alkylhydroxylaminhydrochlorids unter Rühren bei 10 bis 30°C während 5 bis 30 min wird das Alkylhydroxylamin als Base freigesetzt.

Als Alkali- oder Erdalkalihydrogencarbonat sind Natrium-, Kalium-, Magnesium- oder Calciumhydrogencarbonat geeignet.

Das freie Hydroxylamin gibt man als wässrige Lösung unter Rühren bei 40 bis 70°C, vorzugsweise 50 bis 60°C, innerhalb 10 bis 30 min zu der Lösung des Nitrobenzaldehyds in einem inerten organischen Lösungsmittel und rührt 1 bis 4, vorzugsweise 2 bis 3 h bei 50°C nach.

Das Molverhältnis von Ausgangsstoff 2 zu 7 beträgt im Allgemeinen 0,9 bis 1,2, vorzugsweise 0,95 bis 1,1.

An Stelle des O-Alkylhydroxylaminhydrochlorids kann man auch Hydroxylaminhydrochlorid auf analoge Weise in die freie Base überführen und wie beschrieben mit dem Aldehydderivat 2 umsetzen. Das hierbei erhaltene Oxim 4 muss dann noch mit einem Alkylierungsmittel 5 alkyliert werden.

Als Alkylierungsmittel 5 sind Alkylhalogenide, z.B. Alkylchloride, -bromide oder-iodide, Dialkylsulfate oder Arylsulfonsäureester geeignet. Zweckmäßig läßt man das Alkylierungsmittel bei 10 bis 60°C, vorzugsweise 20 bis 40°C, 0,5 bis 5 h, insbesondere 1 bis 2 h, auf das Oxim 5 in Gegenwart einer Base einwirken.

Als Base können die oben erwähnten Hydrogencarbonate, ferner Alkali- oder Erdalkalicarbonate sowie Alkali- und Erdalkalihydroxide verwendet werden. Alkali steht vorzugsweise für Natrium und Kalium, Erdalkali für Magnesium und Calcium.

Das Molverhältnis von 4 zu 5 beträgt im Allgemeinen 0,9 bis 1,4, vorzugsweise 1,1 bis 1,2.

Analoge Alkylierungen sind in Houben-Weyl, Methoden der Organischen Chemie, IV Auflage, Band VI/3, S. 24-37 beschrieben.

Die so erhaltenen Oximether 6 werden anschließend mit Eisen in Gegenwart einer Säure reduziert. Vorteilhaft bringt man den Oximether 6 in einem Gemisch einer Carbonsäure, wie Essigsäure, und einem Alkohol, wie Methanol, mit einem Gemisch von Eisenpulver in dem gleichen Gemisch von Carbonsäure und Alkohol 2 bis 6 h, vorteilhafterweise 3 bis 4 h, bei 70 bis 80°C in Kontakt.

Die Reduktion des Oximethers 6 kann jedoch auch mit Wasserstoff in Gegenwart eines Metallkatalysators bei 10 bis 40°C durchgeführt werden. Man kann die Reduktion auch unter Druck in einem Autoklaven, beispielsweise unter Verwendung von Raney-Nickel, durchführen. Zweckmäßig hydriert man bei 20 bis 80°C, vorteilhaft 40 bis 60°C und 1 bis 50 bar, vorteilhaft 10 bis 20 bar Wasserstoffdruck.

Als Metallkatalysatoren sind Platin, Palladium, Raney-Nickel, Raney-Cobalt oder auch Platinoxid geeignet. Geeignete Hydrierverfahren sind in Houben-Weyl, Methoden der Organischen Chemie, IV.Auflage, Band 11/1, S. 341-359 beschrieben.

Bei den in Schema 2 aufgeführten Reaktionsschritten legt man zweckmäßig das Anilin 8 in Wasser vor, gibt dann nacheinander zunächst portionsweise Hydroxylammoniumsulfat, dann tropfenweise konz. Schwefelsäure und zuletzt Chloral unter Rühren bei 20 bis 40°C hinzu. Man erwärmt 10 bis 30 min auf 50°C und stellt dann durch Zugabe von konz. Natronlauge einen pH von 1,5-2 ein. Nach 6 bis 16 h, vorteilhaft 8 bis 12 h bei Raumtemperatur, isoliert man den gebildeten Niederschlag, nimmt in einer Base auf, wäscht mit einem mit Wasser nicht mischbaren organischen Lösungsmittel und fällt das Isonitrosoacetanilid 9 durch Zugabe einer Säure, z.B. Schwefelsäure.

Im Allgemeinen verwendet man etwa 0,9 bis 1,2, vorzugsweise 0,95 bis 1,05 mol Chloral und 2 bis 4 mol, vorzugsweise 2 bis 3 mol Hydroxylammoniumsulfat pro Mol 7.

Zur Cyclisierung der Isonitrosoacetanilide 9 zu den entsprechenden Isatinen 10 behandelt man die Ausgangsstoffe 9 2 bis 5 h bei 60 bis 90°C, vorteilhaft 70 bis 80°C mit einer starken Säure, z.B. 90%-ige Schwefelsäure.

Die Synthese der Ausgangsstoffe 9 und 10 folgt hierbei den in Beilstein, Band 21, 402-405 beschriebenen Herstellungsmethoden.

Bei den in Schema 3 aufgeführten Reaktionsschritten oxydiert man die Isatine 10 mit Wasserstoffperoxid in Gegenwart von Alkoholen 11 und Alkalialkoholaten 12 zu den Anthranilestern 13. Hierzu gibt man zu den Ausgangsstoffen 10 in einem Gemisch eines Alkohols 9 und seines Alkalialkoholats unter Kühlung wässriges Wasserstoffperoxid hinzu und behandelt 20 bis 60 min, zweckmäßig 30 bis 40 min bei Raumtemperatur.

Die molaren Mengen, in denen die Ausgangsstoffe umgesetzt werden, belaufen sich auf 40 bis 100, insbesondere 60 bis 80 mol Alkohol, 1 bis 5 mol, insbesondere 1 bis 3 mol Alkalialkoholat und 1 bis 3, insbesondere 1 bis 1,5 mol Wasserstoffperoxid pro mol Isatin 10.

Die Herstellung der Anthranilester 13 folgt hierbei dem in der EP 32 672 beschriebenem Verfahren.

Die Isatine 10 lassen sich weiterhin nach der in Schema 1 beschriebenen Weise, ferner auch nach J. Heterocycl. Chem. 1980, 17, 65 in ihre β-Oxime umwandeln.

Erhitzt man diese in Gegenwart von inerten Lösungs- oder Verdünnungsmitteln, deren Siedepunkte nicht unter dem Siedepunkt der entstehenden o-Aminobenzonitrile 15 liegen, auf 200 bis 400°C, vorzugsweise 200 bis 300°C, bei 0,1 bis 200 mbar, vorzugsweise 10 bis 120 mbar, so destillieren die Verbindungen 15 in hoher Reinheit über. Geeignete inerte Lösungs- und Verdünnungsmittel wurden bereits oben genannt.Die Herstellung folgt der in der DE 12 31 709 beschriebenen Verfahrensweise.

Bei den in Schema 4 aufgeführten Reaktionsschritten oxydiert man die Isatine 10 mit Wasserstoffperoxid in einer Carbonsäure, wie Essigsäure, als Reaktionsmedium in Gegenwart katalytischer Mengen Schwefelsäure zunächst zu den Isatosäureanhydriden 16. Hierzu behandelt man die Isatine 10 in Essigsäure mit (pro mol Isatin 10) 4-8 ml, bevorzugt 5-6 ml konz. Schwefelsäure und Wasserstoffperoxid und hält die Reaktionstemperatur bei 50-80°C, bevorzugt 60-70°C. Die molaren Mengen an Wasserstoffperoxid pro mol Isatin 10 betragen 0,95 bis 1,3, bevorzugt 1,0 bis 1,15 mol.

Die Herstellung folgt der in Angew. Chem. 1980, 92, 196 beschriebenen Reaktionsweise.

Zur Überführung der Isatosäureanhydride 16 in die Anthranilester 13 suspendiert man die Ausgangsstoffe 16 in überschüssigem Alkohol als Reaktionsmedium, gibt pro mol Ausgangsstoff 16 0,6 bis 1 mol, bevorzugt 0,7 bis 0,9 mol einer organischen Base, wie Triethylamin, Tri-n-propylamin, Cyclohexyldimethylamin oder N-Methylmorpholin, hinzu und behandelt 1 bis 5 h, bevorzugt 2 bis 3 h, bei 50 bis 80°C, bevorzugt 60 bis 70°C. Die Gewinnung der Anthranilester 13 erfolgt in üblicher Weise. Statt der obengenannten organischen Basen kann man auch 1 bis 10, bevorzugt 2 bis 5 mol% einer hochnucleophilen organischen Base, wie 4-Dimethylaminopyridin, als Katalysator verwenden.

Die Arbeitsweise folgt der in der WO 97/08130 und J. Med. Chem. 1988, 31, 2136 beschriebenen Verfahrensweise.

Statt mit Alkoholen kann die Ringöffnung der Isatosäureanhydride 16 auch mit Ammoniak zu den Anthranilamiden 17 erfolgen. Hierzu wird wässriger Ammoniak und Verbindung 16 in einem polaren wässrigen Lösungsmittel, wie Dimethylformamid, Dimethylacetamid oder N-Methylpyrrolidon, bei 70 bis 95°C, vorteilhaft 80 bis 90°, in Kontakt gebracht. Die Menge an Ammoniak, bezogen auf den Ausgangsstoff 16 beträgt 0,95 bis 1,3, bevorzugt 1,1 bis 1,2 mol.

Die Arbeitsweise folgt der in DOS 1543 332 beschriebenen Verfahrensweise.

Zur Überführung der Anthranilamide 17 in die entsprechenden Benzonitrile 15 behandelt man die Ausgangsstoffe 17 zweckmäßig in Form des Hydrochlorids 1 bis 8 h, vorteilhaft 3 bis 4 h bei 110 bis 150°C, bevorzugt 120 bis 130°C mit wasserentziehenden Mitteln, wie Phosphoroxychlorid. Das Verfahren folgt den in J. Chem. Soc. Chem. Comm., 1994, (15), 1767, J. Heterocycl. Chem. 1997, 34, 1661 beschriebenen Verfahrensweisen.

Überraschenderweise wurde gefunden, daß die Herstellung der erfindungsgemäßen Verbindungen, bei denen R²¹ für CN steht, durch Behandlung der entsprechenden Carbamoylverbindungen, d.h. Verbindungen der Formel 1, bei der R²¹ für CONH₂ steht, mit Phosphoroxychlorid glatt und in hoher Ausbeute erfolgt. Im Hinblick auf den Stand der Technik, z.B. Houben-Weyl, Methoden der Organischen Chemie, 4. Auflage, Band 12/2, Seiten 387 und 448 und Band E2, S. 527, hätte man die Bildung von Phosphorsäureanilidderivaten erwartet. Die Ausgangsverbindungen setzt man dabei zweckmäßig in Form eines Salzes mit einer anorganischen oder organischen Säure ein. Geeignete Säuren sind z.B. Salzsäure, Bromwasserstoffsäure, Schwefelsäure, Phosphorsäure, Salpetersäure, Essigsäure oder Propionsäure. Bevorzugt ist Salzsäure.

Das Phosphoroxychlorid wird im Allgemeinen im Überschuß verwendet. Die Reaktionsbedingungen sind oben im Zusammenhang mit den Benzonitrilen 15 angegeben.

Bei den in Schema 5 aufgeführten Reaktionsschritten schützt man die Anthranilester 13 mit einem Acylrest. Geeignete Acylierungsmittel sind Chloride oder Bromide der Essigsäure, Propionsäure, Buttersäure, Isobuttersäure oder Valeriansäure, ferner Anhydride dieser Säuren. Es können auch gemischte Anhydride, beispielsweise Formylacetat eingesetzt werden. Zweckmäßig läßt man das Acylierungsmittel bei Verwendung eines Anhydrids bei 20 bis 140°C, vorteilhaft 80 bis 120°C, innerhalb 4 bis 20 h, vorteilhaft 6 bis 12 h auf den Anthranilester in einem inerten Lösungsmittel einwirken. Bei Verwendung von Säurechloriden als Acylierungsagens kontaktiert man das Säurechlorid mit einer Mischung des Anthranilesters 13 und einer Base in einem inerten Lösungsmittel bei 10 bis 60°C, vorteilhaft 20 bis 30°C, während 2 bis 20 h, vorteilhaft 6 bis 12 h. Als Base können die vorgenannten organischen und anorganischen Basen, ferner Pyridin, α-, β-, γ-Picolin, Lutidin, Chinolin oder Acridin verwendet werden. Bei Verwendung von Säurechloriden oder -bromiden kann man auch in einem Zweiphasensystem arbeiten, das sich bei Verwendung von Wasser bildet. Die Acylierung kann bei Verwendung von Anhydriden als Acylierungsreagens auch durch hochnucleophile Basen, wie p-Dimethylaminopyridin oder p-Pyrrolidinopyridin, katalytisch beschleunigt werden. Die molaren Verhältnisse in denen die Ausgangsstoffe miteinander umgesetzt werden, liegen bei 0,95 bis 1,3, vorteilhaft 1,0 bis 1,1 mol Acylierungsagens und Base pro mol Anthranilester 13. Den Katalysator setzt man zweckmäßig in einer Menge von 0,5 bis 1,0, vorteilhaft 1 bis 3 mol% pro mol Anthranilester 13 ein.

Zur Reduktion des acylierten Anthranilesters 19 bringt man diesen in einem inerten Lösungsmittel mit einem komplexen Metallhydrid, wie Natriumboranat, in einem der vorgenannten Lösungsmittel bei 10 bis 65°C, vorteilhaft 20 bis 50°C 2 bis 10 h, vorteilhaft 3 bis 6 h in Kontakt. Geeignete inerte Lösungsmittel sind Acetonitril oder wässrige Alkohole (bei Verwendung von Natriumboranat) oder Diisopropylether oder Tetrahydrofuran (bei Verwendung von Lithiumaluminiumhydrid oder Lithiumboranat).

Die molaren Verhältnisse, in denen die Ausgangsstoffe miteinander umgesetzt werden, liegen bei 0,5 bis 3, vorteilhaft 0,75 bis 2,5 mol Lithiumboranat pro mol Ausgangsstoff 19.

Man kann die Ausgangsstoffe 19 auch durch Behandlung mit 0,95 bis 1,1 mol, vorteilhaft 1,0 bis 1,03 mol wässrigem Alkali bei 10 bis 80°C, vorteilhaft 20 bis 60°C, während 1 bis 10 h, vorteilhaft 2 bis 6 h an der Estergruppe verseifen und dann wie oben mit einem komplexen Metallhydrid reduzieren. Das Verfahren folgt der in Organikum, VEB Deutscher Verlag der Wissenschaften, Berlin 1976, 15. Auflage, S. 612-616 beschriebenen Verfahrensweise.

Anschließend werden die Benzylalkohole 21 durch Behandlung mit einem Alkylierungsmittel 5 alkyliert. Die Reaktion wird unter den gleichen Reaktionsbedingungen wie für die Alkylierung des Oxims 4 gemäß Schema 1 durchgeführt.

Zur Freisetzung des o-Alkoxymethylanilins 23 wird Verbindung 22 mit wässrigem Alkali, zweckmäßig 0,95 bis 1,2 mol, vorteilhaft 1,0 bis 1,1 mol, 1 bis 12 h bei 20 bis 120°C, vorteilhaft 2 bis 8 h bei 60 bis 100°C hydrolysiert.

Bei den in Schema 6 aufgeführten Reaktionsschritten unterzieht man die geschützten Aniline 19 einer Chlorierung an ihrer Tolylseitenkette. Man kann hierzu elementares Chlor und eine Apparatur zur kontinuierlichen Chlorierung, wie für die Chlorierung von Toluol zu Benzylchlorid in Houben-Weyl, Methoden der Organischen Chemie, 4. Auflage, Band 5/3, S. 520 beschrieben, verwenden. Man kann jedoch auch N-Chlor- oder N-Bromverbindungen mit positiv induziertem Halogen verwenden, wie ebenda S. 800 beschrieben. Beispielsweise wird auf S. 800-801 die Chlorierung von Toluol mit N-Chlorsuccinimid unter Belichtung oder unter Zugabe von Peroxiden zur Seitenkettenchlorierung näher beschrieben.

Ebenda, S. 807 erläutert die Chlorierung der Tolylseitenkette mit 1,3-Dichlor-5,5-dimethyl-hydantoin. Statt elementarem Halogen kann man auch das milder wirkende Sulfurylchlorid verwenden und die Reaktion durch Zugabe eines Radikalstarters, wie Azoisobutyronitril oder Benzoylperoxid, katalysieren. Die Reaktion folgt der in Houben-Weyl, Band V/3, S. 892 beschriebenen Verfahrensweise.

Als Lösungsmittel eignen sich höher chlorierte Kohlenwasserstoffe wie Dichlor- und Trichlorbenzol, Chloroform, insbesondere Tetrachlorkohlenstoff, ferner Acetonitril oder Essigsäure. Man kann jedoch auch ohne Lösungsmittel arbeiten und direkt in eine Schmelze des Ausgangsstoffes 19 Chlor oder Sulfurylchlorid einleiten.

Die Menge an Chlorierungsreagens beträgt 0,7 bis 1,5, zweckmäßig 0,95 bis 1,1 mol Chlorierungsreagenz pro mol Ausgangsstoff 19. Je nach Chlorierungsreagens arbeitet man bei 10 bis 200°C, vorteilhaft 20 bis 150°C während 10 min bis 10 h, vorteilhaft 0,5 bis 6 h.

Zur Überführung der Benzylchloride 24 in ihre Alkoxymethylether bringt man ein Alkanol 11 und zweckmäßig dessen Alkoholat 12 mit 14 bei 10 bis 100°C, vorteilhaft 20 bis 80°C 0,5 bis 8 h, vorteilhaft 1 bis 4 h in Kontakt. Statt des Alkoholats 12 kann man auch eine der oben erwähnten Basen oder ein Alkalihydroxid in dem betreffenden Alkohol einsetzen.

Die molaren Mengen, in denen das Alkoholat 12 oder die Base eingesetzt wird, betragen 0,95 bis 1,2, vorteilhaft 1,0 bis 1,1 mol pro mol Benzylchlorid 24.

Das Verfahren ist in Houben-Weyl, 4. Auflage, Band 6/3, S. 24-32 beschrieben.

Zur Freisetzung der Verbindungen 23 werden die Ausgangsstoffe 22 mit wässrigem Alkali (zweckmäßig 0,95 bis 1,2 mol, vorteilhaft 1,0 bis 1,1 mol) 1 bis 20 h, vorteilhaft 2 bis 10 h bei 20 bis 120°C, vorteilhaft 70 bis 100°C behandelt.

Alle oben beschriebenen Reaktionsschritte können drucklos oder unter Druck, kontinuierlich oder diskontinuierlich durchgeführt werden.

Die Konzentration der Edukte im Lösungsmittel beträgt 0,1 bis 5 mol/l, bevorzugt 0,2 bis 2 mol/l.

Die Aufarbeitung der Reaktionsgemische erfolgt in der Regel nach an sich bekannten Methoden, beispielsweise durch Verdünnen der Reaktionslösung mit Wasser und anschließender Isolierung des Produktes mittels Filtration, Kristallisation oder Lösungsmittelextraktion, oder durch Entfernen des Lösungsmittels und Destillation oder Verteilen des Rückstandes in einem Gemisch aus Wasser und einem geeigneten organischen Lösungsmittel und Aufarbeiten der organischen Phase auf das Produkt hin.

Soweit nicht anders angegeben, verwendet man als Lösungsmittel für die obigen Umsetzungen - je nach Temperaturbereich - Kohlenwasserstoffe wie Pentan, Hexan, Cyclopentan, Cyclohexan, Toluol, Xylol, chlorierte Kohlenwasserstoffe wie Methylenchlorid, Chloroform, 1,2-Dichlorethan, 1,1,2,2-Tetrachlorethan, Chlorbenzol, 1,2-, 1,3- oder 1,4-Dichlorbenzol, Ether wie 1,4-Dioxan, Tetrahydrofuran, Anisol, Glycolether wie Dimethylglycolether, Diethylglycolether, Diethylenglycoldimethylether, Ester wie Ethylacetat, Propylacetat, Methylisobutyrat, Isobutylacetat, Carbonsäureamide wie Dimethylformamid, N-Methylpyrrolidon, Nitrokohlenwasserstoffe wie Nitrobenzol, Harnstoffe wie Tetraethylharnstoff, Tetrabutylharnstoff, Dimethylethylenharnstoff, Dimethylpropylenharnstoff, Sulfoxide wie Dimethylsulfoxid, Sulfone wie Dimethylsulfon, Diethylsulfon, Tetramethylensulfon, Nitrile wie Acetonitril, Propionitril, Butyronitril oder Isobutyronitril; Wasser oder auch Gemische einzelner Lösungsmittel.

Die Anilinverbindungen sind in guten Ausbeuten zugänglich. Sie lassen sich auch in größerem Maßstab herstellen. Sie sind daher in besonderem Maße als Ausgangsprodukte für die Herstellung von Verbindungen der allgemeinen Formel A 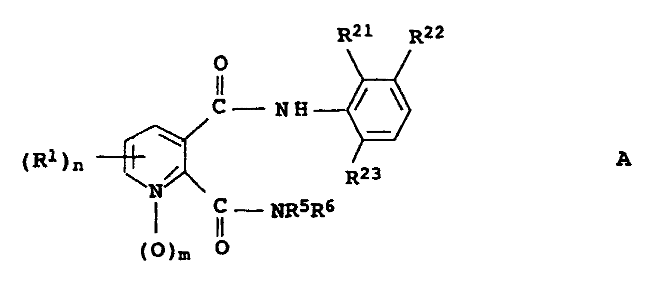 geeignet, worin R¹, R⁵, R⁶, R²¹, R²², R²³, m und n die oben angegebenen Bedeutungen besitzen.

Die Weiterverarbeitung der Anilinverbindungen in die Verbindungen der Formel A kann nach an sich bekannten Verfahren der Literatur erfolgen, z.B. analog den in EP 799 825 beschriebenen Synthesewegen. Der Inhalt der EP 799 825 ist Bestandteil der vorliegenden Offenbarung.

Das bevorzugte Weiterverarbeitungsverfahren umfaßt die Umsetzung einer Pyridin-2,3-dicarbonsäureanhydridverbindung der Formel B 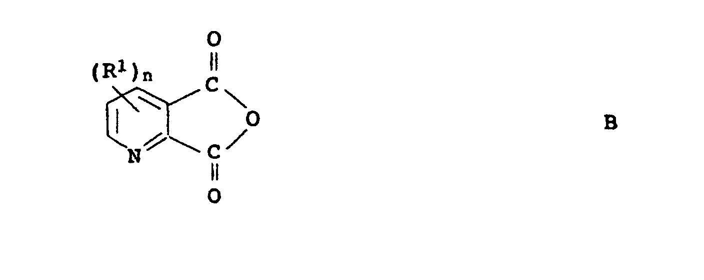 mit einem Anilin der Formel 1. Die Umsetzung führt zu einer Verbindung der Formeln C oder D 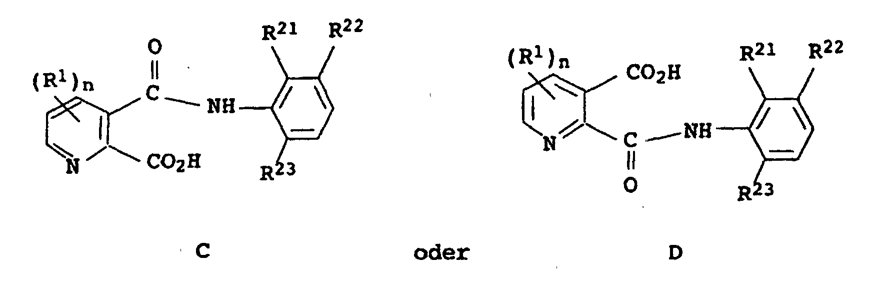 oder einem Gemisch davon. Sie erfolgt im Allgemeinen in einem inerten Lösungsmittel, z.B. einem chlorierten Lösungsmittel, wie Dichlormethan oder 1,2-Dichlorethan, einem aromatischen Kohlenwasserstoff, wie Toluol oder Xylol oder einem Ether, wie Diethylether, Dioxan oder Tetrahydrofuran. Die Umsetzung kann in einem weiten Temperaturbereich, z.B. von Raumtemperatur bis zum Siedepunkt des Lösungsmittels erfolgen.

Das Reaktionsprodukt wird mit einem Dehydratisierungsmittel, wie Acetanhydrid oder Thionylchlorid, mit oder ohne inertem Lösungsmittel, zu einem Imid der Formel E 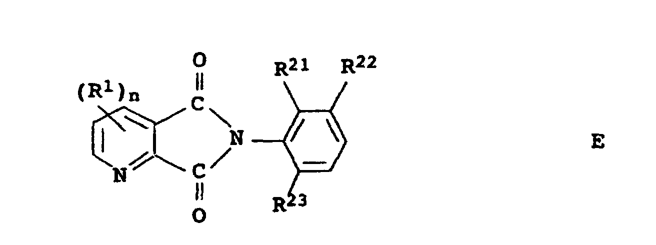 cyclisiert. Als inertes Lösungsmittel kommen die oben angegebenen Lösungsmittel in Betracht. Alternativ kann die Cyclisierung durch Erhitzen (1 bis 20 h) der Verbindungen C und/oder D in der Schmelze bevorzugt bei einer Temperatur im Bereich von 150°C bis 250°C, erfolgen.

Das Imid wird anschließend mit einem Amin der Formel HNR⁵R⁶ zu der entsprechenden Verbindung der Formel A umgesetzt (R¹, R⁵, R⁶ und n besitzen die oben angegebenen Bedeutungen). Die Reaktionsbedingungen für die obigen Reaktionsschritte sind detailliert in der EP-A-799 825 beschrieben. Falls eine Verbindung der Formel A mit m=1 erwünscht ist, wird eine Oxidation auf der Stufe des Imids mit einem geeigneten Oxidationsmittel, wie Wasserstoffperoxid oder organische Persäuren, z.B. Peressigsäure, m-Chlorperbenzoesäure, vorgenommen, siehe EP-A-799 825. Die Pyridin-2,3-dicarbonsäureanhydride sind nach bekannten Verfahren herstellbar, beispielsweise durch Behandlung der Pyridin-2,3-dicarbonsäuren mit Phosgen in Gegenwart von Dimethylformamid nach dem in der US 4,439,607 beschriebenen Verfahren.

Weitere Pyridindicarbonsäure-Ausgangsmaterialien sind in EP 227 932, 322 616, 461 403, 422 456, 661 282 und 663 399 beschrieben oder können nach den dort beschriebenen Methoden hergestellt werden. Die Amine der Formeln Q-NH₂ und HNR⁵R⁶ sind bekannt oder können nach dem Fachmann bekannten Verfahren hergestellt werden.

Die nachfolgenden Beispiele erläutern die Erfindung ohne sie zu begrenzen.

### Beispiel 1

### 3-Chlor-2-methoxyiminomethyl-anilin

a) 3-Chlor-2-methoxyiminomethyl-anilin-nitrobenzol
   25,4 g (0,302 mol) Natriumhydrogencarbonat wurden portionsweise unter Rühren zu einer Mischung von 126 g (0,302 mol) 20%-igem o-Methylhydroxylamin-hydrochlorid in 140 ml Wasser innerhalb von 10 min bei 22°C gegeben. Anschließend wurde diese Lösung bei 50 bis 55°C unter Rühren innerhalb 30 min zu 50 g 2-Chlor-6-nitrobenzaldehyd in 270 ml Toluol gegeben und 2 h bei 50°C nachgerührt. Nach dem Abkühlen gab man 250 ml Wasser und 250 ml Toluol zu dem Reaktionsgemisch zur Trennung der Phasen. Die wässrige Phase wurde noch einmal mit Toluol extrahiert. Die organischen Auszüge wurden mit Wasser und verdünnter Kochsalzlösung gewaschen, über Magnesiumsulfat getrocknet und im Vakuum eingeengt. Man erhielt 52 g (84% d.Th.) der Titelverbindung als gelbliches Öl mit n_{D}²³ = 1,5691.
b) 3-Chlor-2-methoxyiminomethyl-anilin
   35,1 g (0,629 mol) Eisenpulver wurden in einem Gemisch von 100 ml Essigsäure und 140 ml Methanol vorgelegt und auf 70°C erwärmt. Nun wurden unter Rühren bei 70 bis 75°C innerhalb 45 min 45 g (0,21 mol) der Verbindung aus 1a) in 100 ml Essigsäure und 140 ml Methanol zugegeben und 3 h bei 70°C nachgerührt. Nach dem Abkühlen wurde die Suspension auf 3 l Wasser gegossen und mit 0,5 l Essigester verrührt. Nach dem Absaugen wurde der Niederschlag mit 0,5 l Essigester gewaschen und die Phasen getrennt. Die wässrige Phase wurde noch 2 x mit Essigester extrahiert, die organischen Auszüge vereinigt, über Magnesiumsulfat getrocknet und im Vakuum eingeengt. Man erhielt 38.7 g (95% d.Th.) der Titelverbindung als gelbliches Öl mit n_{D}²³ = 1,6140.

### Beispiel 2

### 2-Amino-6-chlor-3-methylbenzoesäuremethylester

110 g (0,52 mol) 6-Chlor-3-methyl-isatosäureanhydrid wurden bei 22°C unter Rühren in eine Mischung von 750 ml Methanol und 47,6 g (0,47 mol) Triethylamin eingetragen und dann 2 h bei 65°C gerührt. Das Reaktionsgemisch wurde im Vakuum eingeengt, in Methylenchlorid aufgenommen und 3 x mit 0,5 n Natronlauge extrahiert. Nach dem Trocknen über Magnesiumsulfat, Filtrieren über Kieselgel und Einengen im Vakuum erhielt man 45,5 g (43,8% d.Th.) der Titelverbindung als farbloses Öl mit n_{D}²³ = 1,5765.

### Beispiel 3

### 2-Amino-3-chlor-6-methylbenzamid

Eine Lösung von 59 ml (0,78 mol) 25%-ige Ammoniaklösung in 207 ml Wasser und eine Mischung 135,2 g (0,64 mol) 3-Chlor-6-methylisatosäureanhydrid in 705 ml DMF wurden gleichzeitig über 2 Tropftrichter innerhalb 30 min unter Rühren bei 85 bis 90°C zu 150 ml Wasser gegeben, wobei starke Gasabspaltung eintrat. Es wurde 2 h bei 90°C und 10 h bei 22°C nachgerührt. Die Reaktionslösung wurde im Vakuum eingeengt, der Rückstand mit Methyl-tert.-butylether verrührt, abgesaugt und getrocknet. Man erhielt 67,4 g (57% d.Th.) der Titelverbindung als gelbliches Pulver vom Fp. 124-128°C.

### Beispiel 4

### 2-Amino-3-chlor-6-methylbenzonitril

1,18 g (0,033 mol) Chlorwasserstoff, gelöst in 35 ml Diethylether, wurden unter Rühren zu einer Suspension von 5 g (0,027 mol) der Verbindung aus Beispiel 3 in 50 ml 1,2-Dichlorethan bei 22 bis 30°C gegeben und dann im Vakuum eingeengt. Der Rückstand wurde mit 150 ml Phosphoroxychlorid versetzt und 3 h bei 120°C gerührt. Anschließend wurde die Reaktionsmischung im Vakuum eingeengt, in Methylenchlorid gelöst, mit Wasser versetzt und mit 2 n Natronlauge neutralisiert. Nach der Phasentrennung wurde nochmals mit Wasser, dann mit gesättigter Kochsalzlösung gewaschen und über Magnesiumsulfat getrocknet. Nach dem Einengen erhielt man 2,4 g (48% d.Th.) der Titelverbindung als gelbliches Pulver vom Fp. 77-80°C.

### Beispiel 5

### 2-Amino-4-chlor-3-methylbenzamid

Bei 80°C ließ man gleichzeitig eine Suspension von 4,64 g (0,0219 mol) 3-Methyl-4-chlor-isato-säureanhydrid in 21 ml DMF und 3,1 g (0,0219 mol) 25%-iges Ammoniakwasser in 6 ml Wasser über 2 Zuführungen zu 8 ml Wasser unter Rührer innerhalb 15 min zulaufen. Nach 1 h Rühren bei 80°C wurde abgekühlt und 2 x mit Essigester extrahiert. Die organische Phase wurde über Magnesiumsulfat getrocknet und im Vakuum eingeengt, wobei man 2,61 g (64,4% d.Th.) der Titelverbindung als farblose Kristalle vom Fp. 206-208°C erhielt.

### Beispiel 6

### 2-Amino-4-chlor-3-methylbenzonitril

Nach der Verfahrensweise von Beispiel 4 erhielt man bei der Umsetzung von 5 g (27,1 mmol) der Verbindung aus Beispiel 5, 1,18 g (32,5 mmol) Chlorwasserstoff in 100 ml 1,2-Dichlorethan, dann 150 ml Phosporoxychlorid 2,39 g (52,9% d.Th.) der Titelverbindung als gelbliches Pulver vom Fp. 98-99°C.

### Beispiel 7

### 2-Amino-6-chlor-3-methylbenzamid

Nach der Verfahrensweise von Beispiel 3 erhielt man bei der Umsetzung von 98,8 g (0,467 mol) 6-Chlor-3-methyl-isatosäureanhydrid in 330 ml DMF mit 42 ml (0,560 mol) 25%-iger wässriger Ammoniaklösung in 140 ml Wasser und weiteren 110 ml Wasser 24 g (37% d.Th.) der Titelverbindung als gelbliches Pulver vom Fp. 149-152°C.

### Beispiel 8

### 2-Amino-6-chlor-3-methylbenzonitril

Nach der Verfahrensweise von Beispiel 4 erhielt man bei der Umsetzung von 2,85 g (0,0154 mol) der Verbindung aus Beispiel 7, 0,68 g (0,0185 mol) Chlorwasserstoff in 50 ml 1,2-Dichlorethan, dann 100 ml Phosphoroxychlorid 1,3 g (47,5% d.Th.) der Titelverbindung als gelbliches Pulver von Fp. 95-98°C.

### Beispiel 9

### N-(2-Carbamoyl-5-chlor-6-methyl-phenyl)-3-carboxy-6-methyl-pyridin-2-carbonsäureamid (A) und N-(2-Cyano-5-chlor-6-methyl-phenyl)-6-methyl-pyridin-2,3-dicarbonsäureimid (B)

2,6 g (0,0141 m mol) der Verbindung aus Beispiel 5 wurden zu einer Mischung von 2,3 g (0,0141 mol) 6-Methyl-pyridin-2,3-dicarbonsäureanhydrid und 150 ml 1,2-Dichlorethan gegeben und 15 h bei 83°C gerührt. Anschließend gab man 1,76 g (0,0148 mol) Thionylchlorid hinzu und rührte 16 h bei 83°C. Zu dem Reaktionsgemisch gab man 200 ml Methylenchlorid und 150 ml Wasser und trennte die Phasen. Der unlösliche Rückstand wurde abgesaugt und getrocknet, wobei man 0,91 g (18,5% d.Th.) der Titelverbindung A als gelbliches Pulver vom Fp. 252°C Zers. erhielt.

Die organische Phase wurde getrocknet und über Kieselgel chromatographiert, wobei man 1,45 g (33% d.Th.) der Titelverbindung B als klebriges Pulver erhielt.
¹H-NMR (270 MHz, d₆-DMSO): δ 8,44 (d/1H), 7,9 (d/1H) Pyr; 8,0 (d/1H), 7,8 (d/1H) Ph; 2,3 (s/3H) CH₃

### Beispiel 10

### 3-(2-Cyano-5-chlor-6-methyl-phenyl)-aminocarbonyl-6-methyl-pyridin-2-carbonsäure-N-n-propylamid

0,28 g (4,8 mmol) n-Propylamin wurden zu einer Mischung von 0,5 g (1,604 mmol) der Verbindung B aus Beispiel 9 und 20 ml THF gegeben und 14 h bei 22°C gerührt. Das Reaktionsgemisch wurde im Vakuum eingeengt, in Methylenchlorid verrührt und 2x mit 0,5 n Natronlauge gewaschen. Die organische Phase wurde getrocknet und eingeengt, wobei man 0,2 g (30,3% d.Th.) der Titelverbindung als gelbliches Pulver vom Fp 175-176°C erhielt.

### Beispiel 11

### N-(6-Chlor-2-cyano-3-methyl-phenyl)-6-methyl-pyridin-2,3-dicarbonsäureimid

3,1 g (18,61 mmol) der Verbindung aus Beispiel 4 wurden zu 2,9 g (17,7 mmol) 6-Methyl-pyridin-2,3-dicarbonsäureanhydrid gegeben und unter Erwärmen auf 170°C insgesamt 8 h als Schmelze gerührt.

Der Rückstand wurde nach dem Erkalten im Methylenchlorid aufgenommen, und mit Aktivkohle und Magnesiumsulfat versetzt. Nach dem Absaugen wurde über Kieselgel chromatographiert, wobei man nach dem Einengen 1,3 g (24,4% d.Th.) der Titelverbindung als beige Kristalle vom Fp. 173-176°C erhielt.

### Beispiel 12

### 3-Chlor-6-methyl-isatosäureanhydrid

184 g (0,94 mol) 3-Chlor-6-methyl-isatin wurden in eine Mischung von 750 ml Eisessig und 10 ml konz. Schwefelsäure eingetragen und unter Rühren auf 70°C erwärmt. Dann wurden 145 ml (1,279 mol) 30%-iges Wasserstoffperoxid innerhalb 30 min bei gleicher Temperatur zugeführt und 2 h bei 70 bis 75°C gerührt. Der Niederschlag wurde nach dem Abkühlen abgesaugt, mit Wasser gewaschen und getrocknet. Man erhielt 146,6 g (73,7% d.Th.) als beiges Pulver vom Fp. 248-250°C.

### Beispiel 13

### 3-(6-Chlor-2-cyano-3-methyl-phenyl)-aminocarbonyl-6-methyl-pyridin-2-carbonsäure-N-n-propylamid

0,46 g (7,699 mmol) n-Propylamin wurden unter Rühren bei 22°C zu einer Mischung von 0,6 g (1,92 mmol) der Verbindung aus Beispiel 11 in 30 ml Tetrahydrofuran gegeben und es wurde 12 h nachgerührt. Das Reaktionsgemisch wurde im Vakuum eingeengt und der Rückstand mit Diisopropylether/Diethylether 20:1 verrührt. Nach dem Absaugen und Trocknen erhielt man 0,6 g (82,4% d.Th.) der Titelverbindung als farblose Kristalle vom Fp. 129-131°C.

### Beispiel 14

### 3-Chlor-6-methyl-isatin

a) 984 g (6,0 mol) Hydroxylammoniumsulfat wurden portionsweise unter Rühren bei 22°C zu einer Mischung von 343 g (2,0 mol) 2-Chlor-5-methylanilin in 4 1 Wasser gegeben. Nacheinander wurden dann 124,5 g (1,22 mol) 96%-ige Schwefelsäure und 295 g (2,0 mol) Chloral unter Rühren in jeweils 20 min zugetropft und dann wurde 1 h bei 50°C gerührt. Nach dem Abkühlen auf 22°C wurde durch Zugabe von 1,2 l 20%-ige Natronlauge ein pH von 1,8 eingestellt. Nach dem Stehen über Nacht wurde mit Methylenchlorid extrahiert.
b) 3-Chlor-6-methyl-isatin
   36,9 g (0,173 mol) der Verbindung aus 14a wurden in einer Mischung von 185,2 g (1,89 mol) konz. Schwefelsäure und 12,3 g Eis unter Rühren bei 22 bis 70°C eingetragen und 3 h bei 80°C gerührt. Das Reaktionsgemisch wurde auf 30°C abgekühlt und in 3 l Eiswasser eingerührt. Der ausgefallene Niederschlag wurde abgesaugt, mit Wasser gewaschen und getrocknet, wobei man 26,3 g (73% d.Th.) der Titelverbindung vom Fp. 236-238°C erhielt.

### Beispiel 15

### 2-Amino-3-chlor-6-methylbenzoesäuremethylester

28,8 g (0,16 mol) 30%-iges Natriummethylat wurden unter Rühren bei 22°C innerhalb 10 min zu einer Suspension von 25 g (0,128 mol) der Verbindung aus Beispiel 14 b gegeben. Nach Abkühlen auf 0°C wurden dann innerhalb 30 min unter Rühren bei 0 bis 5°C 10,9 g (0,16 mol) 50%-iges Wasserstoffperoxid zugefügt und 1 h bei 22°C nachgerührt. Das Reaktionsgemisch wurde durch Zugabe einer 1-molaren Lösung von Chlorwasserstoff in Ether neutralisiert und im Vakuum eingeengt. Der Rückstand wurde zwischen Methylenchlorid und Wasser verteilt und die organische Phase noch 2 x mit verdünnter Natriunhydrogencarbonatlösung gewaschen. Die organische Phase wurde über Magnesiumsulfat getrocknet, über neutrales Aluminiumoxid gesaugt und eingeengt. Man erhielt 17,7 g (69,4% d.Th.) der Titelverbindung als farbloses Öl mit n_{D}²³ = 1,5761.

## Patentansprüche

1. Substituierte Anilinverbindungen der Formel 1 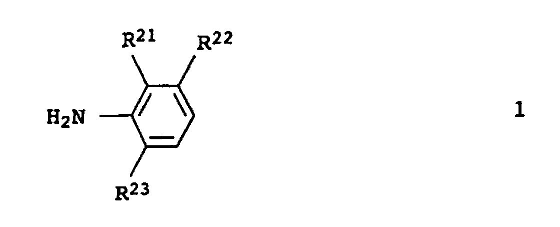 worin die Variablen R²¹, R²² und R²³ die folgenden Bedeutungen haben:
R²¹ C₁-C₃-Alkoxymethyl, C₁-C₃-Alkoximinomethyl, C₁-C₃-Alkoxycarbonyl, CN, C₁-C₃-Alkyl;
R²² Cl, Br, C₁-C₃-Alkyl
R²³ Wasserstoff, C₁-C₃-Alkoxymethyl, C₁-C₃-Alkoxycarbonyl, CN, Cl, Br, C₁-C₃-Alkyl;
wobei
- mindestens einer der Reste R²¹ oder R²³ für C₁-C₃-Alkoxycarbonyl oder ein funktionelles Derivat davon oder für C₁-C₃-Alkoxymethyl steht;
- R²³ nicht Wasserstoff bedeuten kann und R²² und R²³ nicht die gleiche Bedeutung besitzen, wenn R²¹ für C₁-C₃-Alkoxycarbonyl oder CN steht;
- R²² nicht die gleiche Bedeutung wie R²¹ hat, wenn R²¹ für C₁-C₃-Alkyl und R²³ für C₁-C₃-Alkoxycarbonyl stehen, und R²² nicht Cl bedeutet, wenn R²¹ für CH₃ und R²³ für C₁-C₃-Alkoxycarbonyl stehen;
- R²³ nicht für Wasserstoff steht, wenn R²¹ für C₁-C₃-Alkoxymethyl steht,
und die Salze davon,
ausgenommen die Verbindungen der Formel 1, worin
a) R²¹ und R²³ für CN und R²² für CH₃ steht, und
b) R²¹ für CN, R²² für CH₃ und R²³ für CO₂CH₃ steht.

2. Verbindungen der Formel 1 nach Anspruch 1, worin
R²¹ für C₁-C₃-Alkoxymethyl, C₁-C₃-Alkoximinomethyl, C₁-C₃-Alkoxycarbonyl, CN oder C₁-C₃-Alkyl;
R²² für Cl, Br oder C₁-C₃-Alkyl und
R²³ für C₁-C₃-Alkoxymethyl steht.

3. Verbindungen der Formel 1 nach Anspruch 1, worin
R²¹ für C₁-C₃-Alkoxymethyl oder C₁-C₃-Alkoximinomethyl;
R²² für Cl, Br oder C₁-C₃-Alkyl und
R²³ für H, C₁-C₃-Alkoxymethyl, Cl, Br oder C₁-C₃-Alkyl steht.

4. Verbindungen der allgemeinen Formel 1 gemäß Anspruch 1, worin die Variablen R²¹ bis R²³ die folgenden Bedeutungen haben:
R²¹ C₁-C₃-Alkoximinomethyl;
R²² Cl oder Br;
R²³ Wasserstoff.

5. Verbindungen der allgemeinen Formel 1 gemäß Anspruch 1, worin die Variablen R²¹ bis R²³ die folgenden Bedeutungen haben:
R²¹ C₁-C₃-Alkoxycarbonyl oder Cyano;
R²² und R²³, die voneinander verschieden sind, Cl, Br oder C₁-C₃-Alkyl, R²³ ferner C₁-C₃-Alkoxycarbonyl.

6. Verbindungen der allgemeinen Formel 1 gemäß Anspruch 1, worin die variablen R²¹ bis R²³ die folgenden Bedeutungen haben:
R²¹ C₁-C₃-Alkoxymethyl oder C₁-C₃-Alkoximinomethyl
R²² Cl, Br oder C₁-C₃-Alkyl
R²³ C₁-C₃-Alkyl.

7. Verfahren zur Herstellung von substituierten Anilinverbindungen der Formel 1, worin die Reste R²² und R²³ die in Anspruch 1 angegebenen Bedeutungen besitzen und R²¹ für CN steht, **dadurch gekennzeichnet, dass** man eine Verbindung der Formel 25 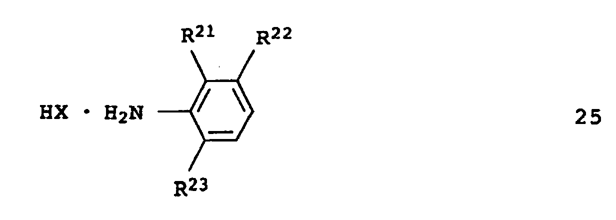 worin die Reste R²² und R²³ die in Anspruch 1 angegebenen Bedeutungen besitzen und R²¹ für CONH₂ steht, und X für das Anion einer anorganischen oder organischen Säure steht, mit Phosphoroxychlorid behandelt.

8. Verfahren zur Herstellung der Verbindungen der Formel A 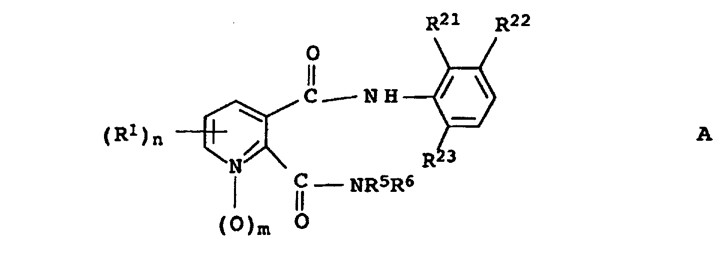 worin die Variablen R²¹, R²² und R²³ die folgenden Bedeutungen haben:
R²¹ C₁-C₃-Alkoxymethyl, C₁-C₃-Alkoximinomethyl, C₁-C₃-Alkoxycarbonyl, CN, C₁-C₃-Alkyl;
R²² Cl, Br, C₁-C₃-Alkyl
R²³ Wasserstoff, C₁-C₃-Alkoxymethyl, C₁-C₃-Alkoxycarbonyl, CN, Cl, Br, C₁-C₃-Alkyl;
wobei
- mindestens einer der Reste R²¹ oder R²³ für C₁-C₃-Alkoxycarbonyl oder ein funktionelles Derivat davon oder für C₁-C₃-Alkoxymethyl steht;
- R²³ nicht Wasserstoff bedeuten kann und R²² und R²³ nicht die gleiche Bedeutung besitzen, wenn R²¹ für C₁-C₃-Alkoxycarbonyl oder CN steht;
- R²² nicht die gleiche Bedeutung wie R²¹ hat, wenn R²¹ für C₁-C₃-Alkyl und R²³ für C₁-C₃-Alkoxycarbonyl stehen, und R²² nicht Cl bedeutet, wenn R²¹ für CH₃ und R²³ für C₁-C₃-Alkoxycarbonyl stehen;
- R³³ nicht für Wasserstoff steht, wenn R²¹ für C₁-C₃-Alkoxymethyl steht,
wobei
a) R²¹ und R²³ nicht für CN stehen, wenn R²² für CH₃ steht, und
b) R²¹ nicht für CN steht, wenn R²² für CH₃ und R²³ für CO₂CH₃ stehen,
R¹ Halogen, CN, NO₂, C₁-C₃-Alkyl, C₁-C₃-Haloalkyl, C₁-C₃-Alkoxy, C₁-C₃-Haloalkoxy, C₁-C₃-Alkylthio, C₁-C₃-Haloalkylthio, C₁-C₃-Alkylsulfinyl, C₁-C₃-Haloalkylsulfinyl, C₁-C₃-Alkylsulfonyl, C₁-C₃-Haloalkylsulfonyl, C₃-C₆-Cycloalkyl, C₃-C₆-Cycloalkyl-C₁-C₃-alkyl, C₂-C₄-Alkenyl, C₂-C₄-Haloalkenyl, C₂-C₄-Alkinyl, C₃-C₄-Haloalkinyl, Amino, C₁-C₃-Monoalkylamino oder C₁-C₃-Alkylcarbonyl;
R⁵ Wasserstoff, C₁-C₃-Alkyl, OH oder C₁-C₄-Alkoxy;
R⁶ Wasserstoff, C₁-C₆-Alkyl, C₁-C₆-Haloalkyl, C₁-C₆-Cyanoalkyl, C₃-C₆-Cycloalkyl, C₃-C₆-Cycloalkenyl, C₃-C₆-Cycloalkyl-C₁-C₃-alkyl, C₃-C₆-Cycloalkyl, das 1 oder 2 Substituenten aufweist, die unabhängig voneinander ausgewählt sind unter Halogen oder C₁-C₃-Alkyl, C₃-C₆-Cycloalkoxy-C₁-C₃-alkyl, C₁-C₆-Alkoxy-C₁-C₆-alkyl, C₁-C₆-Alkylthio-C₁-C₆-alkyl, C₁-C₆-Alkoxycarbonyl-C₁-C₆-alkyl, C₃-C₆-Alkenyl, C₃-C₆-Alkinyl, Amino, C₁-C₄-Monoalkylamino, Di-C₁-C₄-alkylamino oder R⁶ zusammen mit R⁵ ein 5- oder 6-gliedriger Heterocyclus, der 1, 2 oder 3 Heteroatome, die unabhängig voneinander ausgewählt sind unter N, O und S, und gegebenenfalls 1 oder 2 Substituenten aufweist, die unabhängig voneinander ausgewählt sind unter Halogen oder C₁-C₃-Alkyl, C₁-C₃-Alkoxy oder C₁-C₃-Haloalkyl;
m 0 oder 1;
n 0, 1, 2 oder 3,
wobei man eine Verbindung der Formel B 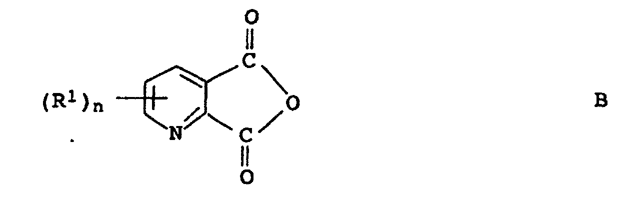 mit einer Verbindung der Formel 1 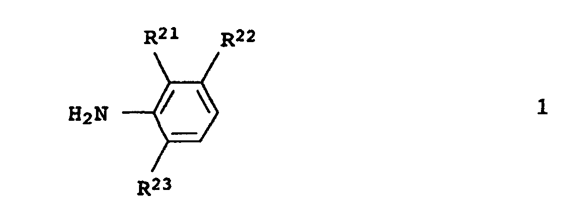 zu einer Verbindung der Formeln C oder D 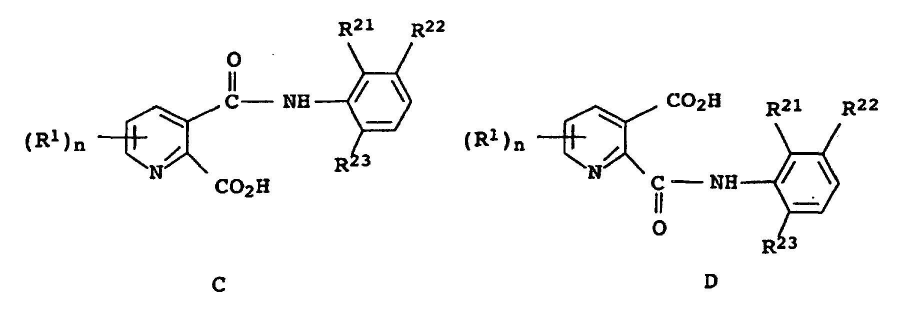 oder einem Gemisch davon umsetzt,
die Verbindung der Formeln C oder D oder das Gemisch davon zu einer Verbindung der Formel E 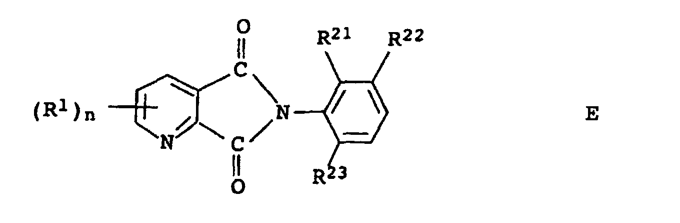 cyclisiert und eine Verbindung der Formel E mit einem Amin der Formel F
HNR⁵R⁶ F
umsetzt.

9. Verwendung der Verbindungen der Formel 1 gemäß einem der Ansprüche 1 bis 6 zur Herstellung einer wie in Anspruch 8 definierten Verbindung der Formel A.

## Claims

1. A substituted aniline compound of the formula 1 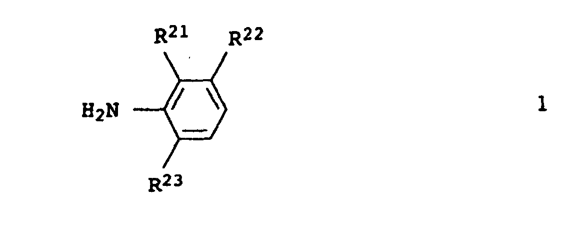 in which the variables R²¹, R²² and R²³ have the following meanings:
R²¹ is C₁-C₃-alkoxymethyl, C₁-C₃-alkoximinomethyl, C₁-C₃-alkoxycarbonyl, CN, C₁-C₃-alkyl;
R²² is Cl, Br, C₁-C₃-alkyl;
R²³ is hydrogen, C₁-C₃-alkoxymethyl, C₁-C₃-alkoxycarbonyl, CN, Cl, Br, C₁-C₃-alkyl;
where
- at least one of the radicals R²¹ or R²³ is C₁-C₃-alkoxycarbonyl or a functional derivative thereof or C₁-C₃-alkoxymethyl;
- R²³ cannot be hydrogen and R²² and R²³ cannot have the same meaning if R²¹ is C₁-C₃-alkoxycarbonyl or CN;
- R²² does not have the same meaning as R²¹ if R²¹ is C₁-C₃-alkyl and R²³ is C₁-C₃-alkoxycarbonyl and R²² is not Cl if R²¹ is CH₃ and R²³ is C₁-C₃-alkoxycarbonyl,
- R²³ is not hydrogen if R²¹ is C₁-C₃-alkoxymethyl,
or the salts thereof,
with the exception of the compounds of the formula 1 in which
a) R²¹ and R²³ are CN and R²² is CH₃, and
b) R²¹ is CN, R²² is CH₃ and R²³ is CO₂CH₃.

2. A compound of the formula 1 as claimed in claim 1, in which
R²¹ is C₁-C₃-alkoxymethyl, C₁-C₃-alkoximinomethyl, C₁-C₃-alkoxycarbonyl, CN or C₁-C₃-alkyl;
R²² is Cl, Br or C₁-C₃-alkyl and
R²³ is C₁-C₃-alkoxymethyl.

3. A compound of the formula 1 as claimed in claim 1, in which
R²¹ is C₁-C₃-alkoxymethyl or C₁-C₃-alkoximinomethyl;
R²² is Cl, Br or C₁-C₃-alkyl and
R²³ is H, C₁-C₃-alkoxymethyl, Cl, Br or C₁-C₃-alkyl.

4. A compound of the general formula 1 as claimed in claim 1, in which the variables R²¹ to R²³ have the following meanings:
R²¹ is C₁-C₃-alkoximinomethyl;
R²² is Cl or Br;
R²³ is hydrogen.

5. A compound of the formula 1 as claimed in claim 1, in which the variables R²¹ to R²³ have the following meanings:
R²¹ is C₁-C₃-alkoxycarbonyl or cyano;
R²² and R²³, which are different from one another, are Cl, Br or C₁-C₃-alkyl, R²³ is furthermore C₁-C₃-alkoxycarbonyl.

6. A compound of the formula 1 as claimed in claim 1, in which the variables R²¹ to R²³ have the following meanings:
R²¹ is C₁-C₃-alkoxymethyl or C₁-C₃-alkoximinomethyl,
R²² is Cl, Br or C₁-C₃-alkyl,
R²³ is C₁-C₃-alkyl.

7. A process for the preparation of substituted aniline compounds of the formula 1 in which the radicals R²² and R²³ have the meanings indicated in claim 1 and R²¹ is CN, which comprises treating a compound of the formula 25 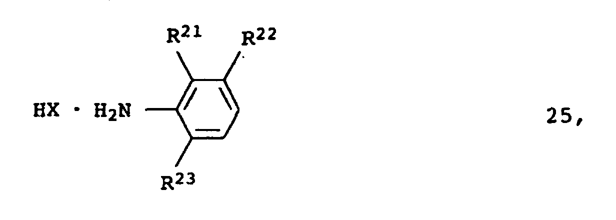 in which the radicals R²² and R²³ have the meanings indicated in claim 1 and R²¹ is CONH₂, and X is the anion of an inorganic or organic acid, with phosphorus oxychloride.

8. A process for the preparation of the compounds of the formula A 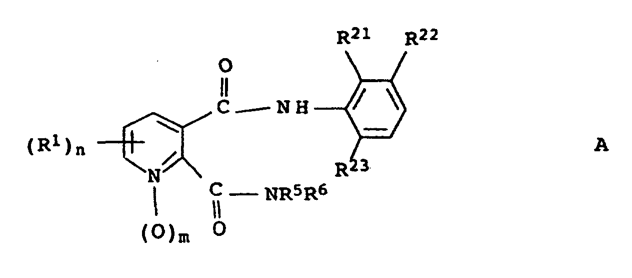 where the variables R²¹, R²² and R²³ have the following meanings:
R²¹ is C₁-C₃-alkoxymethyl, C₁-C₃-alkoximinomethyl, C₁-C₃-alkoxycarbonyl, CN, C₁-C₃-alkyl;
R²² is Cl, Br, C₁-C₃-alkyl;
R²³ is hydrogen, C₁-C₃-alkoxymethyl, C₁-C₃-alkoxycarbonyl, CN, Cl, Br, C₁-C₃-alkyl;
where
- at least one of the radicals R²¹ or R²³ is C₁-C₃-alkoxycarbonyl or a functional derivative thereof or C₁-C₃-alkoxymethyl;
- R²³ cannot be hydrogen and R²² and R²³ cannot have the same meaning if R²¹ is C₁-C₃-alkoxycarbonyl or CN;
- R²² does not have the same meaning as R²¹ if R²¹ is C₁-C₃-alkyl and R²³ is C₁-C₃-alkoxycarbonyl, and R²² is not Cl if R²¹ is CH3 and R²³ is C₁-C₃-alkoxycarbonyl;
- R²³ is not hydrogen if R²¹ is C₁-C₃-alkoxymethyl,
provided that
a) R²¹ and R²³ are not CN if R²² is CH₃, and
b) R²¹ is not CN if R²² is CH₃ and R²³ is CO₂CH₃,
R¹ is halogen, CN, NO₂, C₁-C₃-alkyl, C₁-C₃-haloalkyl, C₁-C₃-alkoxy, C₁-C₃-haloalkoxy, C₁-C₃-alkylthio, C₁-C₃-haloalkylthio, C₁-C₃-alkylsulfinyl, C₁-C₃-haloalkylsulfinyl, C₁-C₃-alkylsulfonyl, C₁-C₃-haloalkylsulfonyl, C₃-C₆-cycloalkyl, C₃-C₆-cycloalkyl-C₁-C₃-alkyl, C₂-C₄-alkenyl, C₂-C₄-haloalkenyl, C₂-C₄-alkynyl, C₃-C₄-haloalkynyl, amino, C₁-C₃-monoalkylamino or C₁-C₃-alkylcarbonyl;
R⁵ is hydrogen, C₁-C₃-alkyl, OH or C₁-C₄-alkoxy;
R⁶ is hydrogen, C₁-C₆-alkyl, C₁-C₆-haloalkyl, C₁-C₆-cyancalkyl, C₃-C₆-cycloalkyl, C₃-C₆-cycloalkenyl, C₃-C₆-cycloalkyl-C₁-C₃-alkyl, C₃-C₆-cycloalkyl which has 1 or 2 substituents which independently of one another are selected from halogen or C₁-C₃-alkyl, C₃-C₆-cycloalkoxy-C₁-C₃-alkyl, C₁-C₆-Alkoxy-C₁-C₆-alkyl, C₁-C₆-alkylthio-C₁-C₆-alkyl, C₁-C₆-alkoxycarbonyl-C₁-C₆-alkyl, C₃-C₆-alkenyl, C₃-C₆-alkynyl, amino, C₁-C₄-monoalkylamino, di-C₁-C₄-alkylamino or R⁶, together with R⁵, is a 5- or 6-membered heterocycle which has 1, 2 or 3 heteroatoms, which independently of one another are selected from N, O and S, and optionally 1 or 2 substituents which independently of one another are selected from halogen or C₁-C₃-alkyl, C₁-C₃-alkoxy or C₁-C₃-haloalkyl;
m is 0 or 1;
n is 0, 1, 2 or 3,
where a compound of the formula B 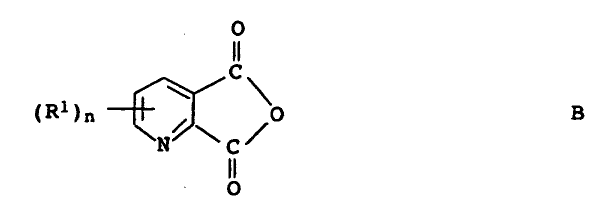 is reacted with a compound of the formula 1 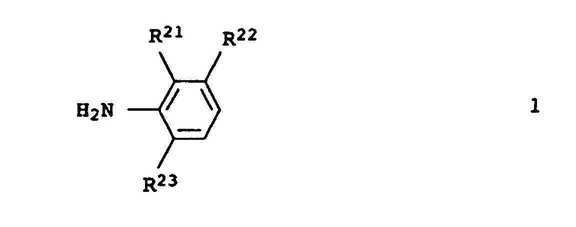 to give a compound of the formula C or D 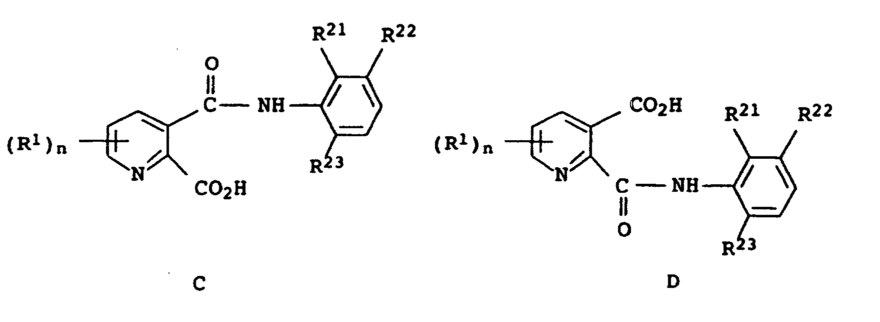 or a mixture thereof,
the compound of the formula C or D or the mixture thereof is cyclized to a compound of the formula E 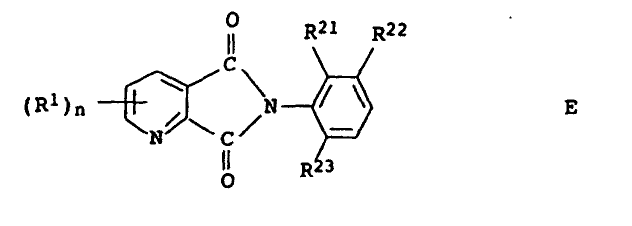 and a compound of the formula E is reacted with an amine of the formula F
HNR⁵R⁶ F.

9. The use of the compounds of the formula 1 as claimed in one of claims 1 to 6 for the preparation of a compound of the formula A as defined in claim 8.

## Revendications

1. Composés d'aniline substitués répondant à la formule 1 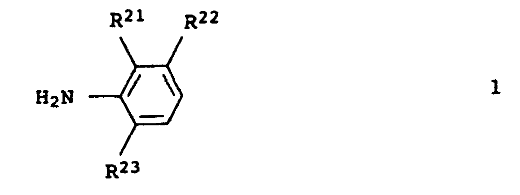 dans laquelle les variables R²¹, R²² et R²³ possèdent les significations suivantes:
R²¹ représente un groupe (alcoxy en C₁-C₃)méthyle, un groupe (alcoxy en C₁-C₃)iminométhyle, un groupe (alcoxy en C₁-C₃)carbonyle, un groupe CN, un groupe alkyle en C₁-C₃ ;
R²² représente Cl, Br, un groupe alkyle en C₁-C₃
R²³ représente un atome d'hydrogène, un groupe (alcoxy en C₁-C₃)-méthyle, un groupe (alcoxy en C₁-C₃)carbonyle, un groupe CN, Cl, Br, un groupe alkyle en C₁-C₃,
dans laquelle
- au moins un des radicaux R²¹ ou R²³ représente un groupe (alcoxy en C₁-C₃)carbonyle ou un dérivé fonctionnel de celui-ci ou un groupe (alcoxy en C₁-C₃)méthyle ;
- R²³ ne peut pas signifier un atome d'hydrogène et R²² et R²³ n'ont pas la même signification, si R²¹ représente un groupe (alcoxy en C₁-C₃)-carbonyle, ou un groupe CN ;
- R²² n'a pas la même signification que R²¹, si R²¹ représente un groupe alkyle en C₁-C₃ et R²³ un groupe (alcoxy en C₁-C₃)carbonyle, et R²² ne signifie pas Cl, si R²¹ représente un groupe CH₃ et R²³ un groupe (alcoxy en C₁-C₃)carbonyle ;
- R²³ ne représente pas un atome d'hydrogène si R²¹ représente un groupe (alcoxy en C₁-C₃)méthyle
et leurs sels,
à l'exception des composés répondant à la formule I, dans laquelle
a) R²¹ et R²³ représentent un groupe CN et R²² un groupe CH₃, et
b) R²¹ représente un groupe CN, R²² un groupe CH₃ et R²³ un groupe CO₂CH₃.

2. Composés répondant à la formule 1 selon la revendication 1, dans laquelle
R²¹ représente un groupe (alcoxy en C₁-C₃)méthyle, un groupe (alcoxy en C₁-C₃)iminométhyle, un groupe (alcoxy en C₁-C₃)carbonyle, un groupe CN ou un groupe alkyle en C₁-C₃ ;
R²² représente Cl, Br ou un groupe alkyle en C₁-C₃ et
R²³ représente un groupe (alcoxy en C₁-C₃)méthyle.

3. Composés répondant à la formule 1 selon la revendication 1, dans laquelle
R²¹ représente un groupe (alcoxy en C₁-C₃)méthyle ou un groupe (alcoxy en C₁-C₃)iminométhyle ;
R²² représente Cl, Br ou un groupe alkyle en C₁-C₃ et
R²³ représente H, un groupe (alcoxy en C₁-C₃)méthyle, Cl, Br ou un groupe alkyle en C₁-C₃.

4. Composés répondant à la formule générale 1 selon la revendication 1, dans laquelle les variables R²¹ à R²³ possèdent les significations suivantes :
R²¹ représente un groupe (alcoxy en C₁-C₃)iminométhyle;
R²² représente Cl ou Br ;
R²³ représente un atome d'hydrogène.

5. Composés répondant à la formule générale 1 selon la revendication 1, dans laquelle les variables R²¹ à R²³ possèdent les significations suivantes :
R²¹ représente un groupe (alcoxy en C₁-C₃)carbonyle ou un groupe cyano;
R²² et R²³, qui sont différents l'un de l'autre, représentent Cl, Br ou un groupe alkyle en C₁-C₃; R²³ signifiant par ailleurs un groupe (alcoxy en C₁-C₃)carbonyle.

6. Composés répondant à la formule générale 1 selon la revendication 1, dans laquelle les variables R²¹ à R²³ possèdent les significations suivantes :
R²¹ représente un groupe (alcoxy en C₁-C₃)méthyle ou un groupe (alcoxy en C₁-C₃)iminométhyle
R²² représente Cl, Br ou un groupe alkyle en C₁-C₃
R²³ représente un groupe alkyle en C₁-C₃.

7. Procédé de production de composés d'aniline substitués répondant à la formule I, dans laquelle les radicaux R²² et R²³ possèdent les significations indiquées à la revendication 1 et R²¹ représente un groupe CN, **caractérisé en ce que** l'on traite avec de l'oxychlorure de phosphore un composé répondant à la formule 25 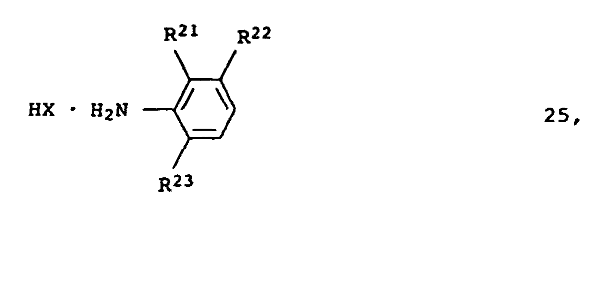 dans laquelle les radicaux R²² et R²³ possèdent les significations indiquées à la revendication 1 et R²¹ représente un groupe CONH₂, et X l'anion d'un acide inorganique ou organique.

8. Procédé de production des composés répondant à la formule A 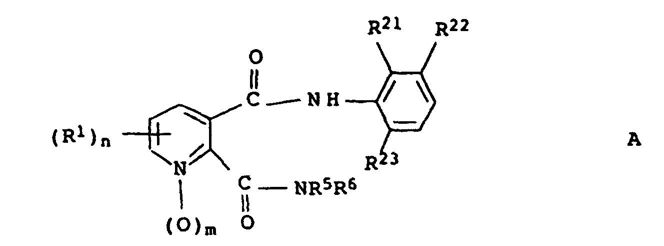 dans laquelle les variables R²¹, R²² et R²³ possèdent les significations suivantes :
R²¹ représente un groupe (alcoxy en C₁-C₃)méthyle, un groupe (alcoxy en C₁-C₃)iminométhyle, un groupe (alcoxy en C₁-C₃)carbonyle, un groupe CN, un groupe alkyle en C₁-C₃ ;
R²² représente Cl, Br, un groupe alkyle en C₁-C₃
R²³ représente un atome d'hydrogène, un groupe (alcoxy en C₁-C₃)méthyle, un groupe (alcoxy en C₁-C₃)carbonyle, un groupe CN, Cl, Br, un groupe alkyle en C₁-C₃ ;
dans laquelle
- au moins un des radicaux R²¹ ou R²³ représente un groupe (alcoxy en C₁-C₃)carbonyle ou un dérivé fonctionnel de celui-ci ou un groupe (alcoxy en C₁-C₃)méthyle ;
- R²³ ne peut pas signifier un atome d'hydrogène et R²² et R²³ n'ont pas la même signification, si R²¹ représente un groupe (alcoxy en C₁-C₃)carbonyle ou un groupe CN ;
- R²² n'a pas la même signification que R²¹, si R²¹ représente un groupe alkyle en C₁-C₃ et R²³ un groupe (alcoxy en C₁-C₃)carbonyle, et R²² ne signifie pas Cl, si R²¹ représente un groupe CH₃ et R²³ un groupe (alcoxy en C₁-C₃)carbonyle;
- R²³ ne représente pas un atome d'hydrogène, si R²¹ représente un groupe (alcoxy en C₁-C₃)méthyle,
où
a) R²¹ et R²³ ne représentent pas un groupe CN, si R²² représente un groupe CH₃, et
b) R²¹ ne représente pas un groupe CN, si R²² représente un groupe CH₃ et R²³ un groupe CO₂CH₃,
R¹ représente un atome d'halogène, un groupe CN, un groupe NO₂, un groupe alkyle en C₁-C₃, un groupe halogénoalkyle en C₁-C₃, un groupe alcoxy en C₁-C₃, un groupe halogénoalcoxy en C₁-C₃, un groupe (alkyle en C₁-C₃)thio, un groupe (halogénoalkyle en C₁-C₃)thio, un groupe (alkyle en C₁-C₃)sulfinyle, un groupe (halogénoalkyle en C₁-C₃)sulfinyle, un groupe (alkyle en C₁-C₃)sulfonyle, un groupe (halogénoalkyle en C₁-C₃)sulfonyle, un groupe cycloalkyle en C₃-C₆, un groupe (cycloalkyle en C₃-C₆)(alkyle en C₁-C₃), un groupe alcényle en C₂-C₄, un groupe halogénoalcényle en C₂-C₄, un groupe alcinyle en C₂-C₄, un groupe halogénoalcinyle en C₃-C₄, un groupe amino, un groupe mono(alkyle en C₁-C₃)amino ou un groupe (alkyle en C₁-C₃)carbonyle;
R⁵ représente un atome d'hydrogène, un groupe alkyle en C₁-C₃, un groupe OH ou un groupe alcoxy en C₁-C₄;
R⁶ représente un atome d'hydrogène, un groupe alkyle en C₁-C₆, un groupe halogénoalkyle en C₁-C₆, un groupe cyanoalkyle en C₁-C₆, un groupe cycloalkyle en C₃-C₆, un groupe cycloalcényle en C₃-C₆, un groupe (cycloalkyle en C₃-C₆)(alkyle en C₁-C₃), un groupe cycloalkyte en C₃-C₆, qui comporte un ou deux substituants, sélectionnés indépendamment les uns des autres parmi un atome d'halogène ou un groupe alkyle en C₁-C₃, un groupe (cycloalcoxy en C₃-C₆)(alkyle en C₁-C₃), un groupe (alcoxy en C₁-C₆)(alkyle en C₁-C₆), un groupe (alkyle en C₁-C₆)thio(alkyle en C₁-C₆), un groupe ((alcoxy en C₁-C₆)carbonyle)-(alkyfe en C₁-C₆), un groupe alcényle en C₃-C₆, un groupe alcinyle en C₃-C₆, un groupe amino, mono(alkyle en C₁-C₄)amino, un groupe di(alkyle en C₁-C₄)amino ou R⁶ conjointement avec R⁵ représente un hétérocycle à 5 ou 6 membres, qui comporte 1, 2 ou 3 hétéroatomes, sélectionnés indépendamment l'un de l'autre parmi N, O et S, et le cas échéant 1 ou 2 substituants, sélectionnés indépendamment l'un de l'autre parmi un atome d'halogène ou un groupe alkyle en C₁-C₃, un groupe alcoxy en C₁-C₃ ou un groupe halogénoalkyle en C₁-C₃ ;
m vaut 0 ou 1 ;
n vaut 0, 1, 2, ou 3,
dans lequel on fait réagir un composé répondant à la formule B 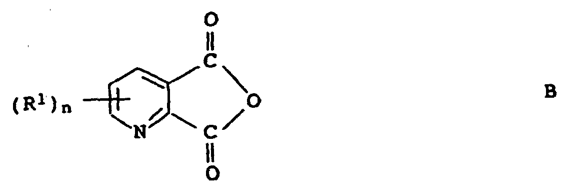 avec un composé répondant à la formule 1 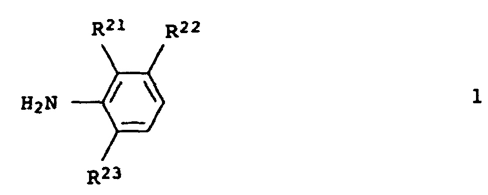 pour donner un composé répondant à la formule C ou D 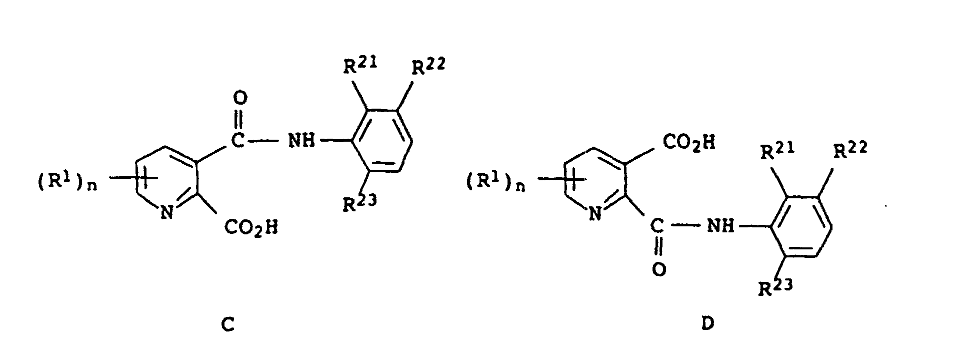 ou un mélange de ceux-ci,
on cyclise le composé répondant aux formules C ou D ou leur mélange pour donner un composé répondant à la formule E 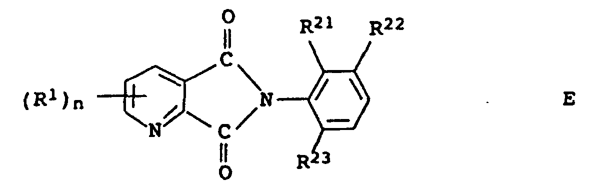 et on fait réagir le composé répondant à la formule E avec une amine répondant à la formule F
HNR⁵R⁶ F

9. Utilisation des composés répondant à la formule 1 selon l'une des revendications 1 à 6 pour la production d'un composé répondant à la formule A comme défini à la revendication 8.
